(19) Europäisches Patentamt
European Patent Office
Office européen des brevets

(11) **EP 1 072 679 A2**

(12) **EUROPEAN PATENT APPLICATION**

(43) Date of publication:
31.01.2001 Bulletin 2001/05

(51) Int. Cl.⁷: **C12N 15/11**, C12P 19/34, C12Q 1/68, C07H 21/00 // C12N9/12

(21) Application number: 00306128.0

(22) Date of filing: 19.07.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 20.07.1999 US 358141

(71) Applicant:
**Agilent Technologies Inc.**
**Santa Clara, California 95052-8043 (US)**

(72) Inventor:
**The designation of the inventor has not yet been filed**

(74) Representative:
**Tollett, Ian et al**
**Williams, Powell & Associates,**
**4 St. Paul's Churchyard Inn**
**London EC4M 8AY (GB)**

(54) **Method of producing nucleic acid molecules with reduced secondary structure**

(57) The present invention provides a system for generating nucleic acid molecules having reduced levels of secondary structure compared to nucleic acid molecules of the same nucleotide sequence containing only naturally-occurring bases. Such molecules are referred to herein as "unstructured nucleic acids" (UNAs). UNAs have reduced levels of secondary structure because of their reduced ability to form intramolecular hydrogen bond base pairs between regions of substantially complementary sequence. Preferred UNAs, however, retain the ability to form intermolecular hydrogen bond base pairs with other nucleic acid molecules.

Figure 1

## Description

### Background of the Invention

Naturally occurring ribonucleic acid (RNA) and deoxyribonucleic (DNA) acid molecules contain bases which are capable of forming base pairs through hydrogen bond interactions with complementary bases, according to the "Watson-Crick" scheme. In naturally occurring RNA, these bases are adenine (rA), uracil (rU), guanine (rG) and cytosine (rC). In naturally occurring DNA, these bases are adenine (dA), thymine (dT), guanine (dG) and cytosine (dC). According to the Watson-Crick scheme, adenine bases in a nucleic acid molecule form base pairs through two hydrogen bonds with thymine bases in DNA (A/T) and uracil bases in RNA (A/U). For G/C base pairs, guanine bases form base pairs through three hydrogen bonds with cytosine bases (G/C) in both DNA and RNA.

Where the nucleotide sequences permit, base pairing can form between two nucleic acid molecules (intermolecular) resulting in a double-helical structure. This can occur between two molecules of DNA, two molecules of RNA or between one molecule of DNA and one molecule of RNA. In addition, base pairs can form between two regions within a single molecule of DNA or RNA (intramolecular) where the two regions contain sequences that permit the formation of base pairs. If two regions of complementarity containing nucleotide sequences between them, hybridization of the two regions to one another results in the formation of a loop. Base pairing hybridization between two regions of complementarity results in a hairpin structure if there are few or no nucleotides between the two regions. In addition, nucleic acid molecules can form three-way junctions and four-way junctions.

Loops, duplexes, hairpin structures, three-way junctions, and four-way junctions are a few examples of secondary structures in nucleic acid molecules. Secondary structure in a nucleic acid molecule can effectively block hybridization of a nucleic acid molecule having substantially complementary sequence. As a result, secondary structure limits the sensitivity, specificity and utility of applications that rely upon hybridization of probes used to analyze nucleic acid molecules of a particular base sequence. Such applications include solution hybridization methods (e.g. mRNA fluorescence in situ hybridization; FISH) and surface-bound oligonucleotide arrays (e.g. DNA array technology) for mutation detection (Chee, M., et al., (1996) *Science* 274, 610-614, Wang, et. al., *Science*, 280, 1077-1082) and gene expression assays (Lockart, D., et al., (1996) *Nat. Biotech.* 14, 1675-1680).

The problem of target secondary structure is particularly acute for those methods that utilize relatively short oligonucleotide probes since the shorter probes are less effective in competing with the intramolecular base-pairing interactions (e.g. US Pat 5,605,798, PCT publications WO92/15712, WO97/35033). These methods include but are not limited to polymerase extension assays (PEA; US Pat 5,595,890; US Pat 5,534,424; US Pat 5,605,798) and oligonucleotide ligation assays (US Pat 4,988,617; US Pat 5,494,810; WO 95/04160).

Solutions to the problem of utilizing oligonucleotide probes to study nucleic acid molecules having secondary structure have been met with only limited success. One attempt to overcome this problem entails performing the hybridization of the oligonucleotide probe to the region having secondary structure in the presence of mild denaturants and/or at elevated temperatures. However, these treatments also result in decreasing the stability of the interaction between the target nucleic acid molecule and the probe (and therefore, overall sensitivity of the assay) due to the decrease hydrogen bond interactions in the entire system.

Although employing longer oligonucleotide probes can compensate for a lowered duplex stability, the use of longer probes can lead to a decrease in sequence specificity which is undesirable. One problem is that the decrease in specificity severely limits the ability of a longer probe to distinguish between polynucleotides that may differ in sequence by only one nucleotide. Since many genetic diseases are caused by single nucleotide mutations, the inability of a longer probe to be highly sequence specific severely limits assays where the goal is to detect single nucleotide changes within the target sequence.

Fragmentation of nucleic acid molecules can also reduce secondary structures that involve intramolecular base pairing between two regions that are separated by large distances (Lockart, D.,.et al., (1996) *Nat. Biotech.* 14, 1675-1680). However, this does not eliminate secondary structure in the form of stable hairpins resulting from short-range base-paring interactions. Moreover, because the detection labels are generally incorporated at multiple internal positions within the target sequence, fragmentation of the target will effectively reduce the target=s overall label specific activity ([label]:[target]) thereby reducing the detection limit of the assay.

Other attempts to reduce the secondary structure in nucleic acid molecules involve the use of chemical modifications to the molecule. For example, methylmercuric hydroxide reacts with the imino bonds of C and G making them unable to base pair with their natural unmodified complements. However, clearly such modifications will also prevent hybridization of the target with its complementary oligonucleotide probe. In addition, chemical modifications of nucleic acids require the use of volatile and toxic reagents which makes the use of this method undesirable.

### Summary of the Invention

The present invention provides a system for the production of nucleic acid molecules with reduced levels of secondary structure. The inventive system allows for the production of nucleic acid molecules with reduced levels of intramolecular base pairing as compared with nucleic acid molecules of substantially identical base sequence comprising all naturally-occurring bases. Preferably, nucleic acid molecules of the present invention having reduced levels of secondary structure are capable of hybridizing to other nucleic acid molecules having substantially complementary base sequences or regions of substantially complementary base sequences.

In one aspect, the present invention provides a method of producing nucleic acid molecules with reduced levels of intramolecular base pairing by incorporating nucleotides having modified bases such that complementary bases in a nucleic acid molecule are unable to form stable hydrogen bond base pairs. A modified base pair may comprise one modified base and one natural base, or it may comprise two modified bases. Preferably, modified bases are positioned in nucleic acid molecules of the present invention in sequence elements of substantially complementary sequence to reduce intramolecular base pairing. Nucleic acid molecules of the present invention are produced by any method.

In a preferred embodiment, nucleic acid molecules of the present invention are produced by incorporating at least one modified purine base and at least one natural pyrimidine base; at least one natural purine base and at least one modified pyrimidine base; or at least one modified purine and at least one pyrimidine bases such that at least one complementary purine/pyrimidine base pair does not form a stable hydrogen bonded base pair. In addition it is preferable that nucleic acid molecules of the present invention are capable of forming at least one stable base pair with at least one substantially complementary sequence element in another nucleic acid molecule.

In another preferred embodiment, nucleic acid molecules of the present invention are produced by incorporating the nucleotides 2-aminoadenosine (D), 2-thiothymidine (2-thioT), inosine (I), pyrrolo-pyrimidine (P), 2-thiocytidine, adenine (A), thymine (T), guanine (G), cytosine (C) and combinations thereof such that at least two complementary bases that are not capable of form a base pair are present.

In yet another preferred embodiment, nucleic acid molecules of the present invention are produced by incorporating the nucleotides 2-aminoadenosine (D), 2-thiothymidine (2-thioT), guanine (G), cytosine (C) and combinations thereof such that 2-aminoadenosine is present in a first sequence element and 2-thiothymidine is present in a second sequence element substantially complementary to the first sequence element.

In yet another preferred embodiment, nucleic acid molecules of the present invention are produced by incorporating the nucleotides inosine (I), pyrrolo-pyrimidine (P), adenine (A), thymine (T), and combinations thereof such that inosine is present in a first sequence element and pyrrolo-pyrimidine is present in a second sequence element substantially complementary to the first sequence element.

In yet another preferred embodiment, nucleic acid molecules of the present invention are produced by incorporating the nucleotides 2-thiocytidine, guanine (G), adenine (A), thymine (T), and combinations thereof such that 2-thiocytidine is present in a first sequence element and guanine is present in a second sequence element substantially complementary to the first sequence element.

In yet another preferred embodiment, nucleic acid molecules of the present invention are produced by incorporating the nucleotides 2-aminoadenosine (D), 2-thiothymidine (2-thioT), inosine (I), pyrrolo-pyrimidine (P), and combinations thereof such that complementary sequence elements are unable to form stable base pairs.

In yet another preferred embodiment, nucleic acid molecules of the present invention are produced by incorporating the nucleotides 2-aminoadenosine (D), 2-thiothymidine (2-thioT), 2-thiocytidine, guanine (G), and combinations thereof such that complementary sequence elements are unable to form stable base pairs.

In yet another preferred embodiment, nucleic acid molecules with reduced levels of secondary structure are produced by chemical synthesis or enzymatic synthesis. Preferably, nucleic acid molecules of the present invention are produced enzymatically.

In yet another preferred embodiment, nucleic acid molecules with reduced levels of secondary structure are produce by an RNA polymerase, a DNA polymerase, a reverse transcriptase, a ribozyme or a self-replicating RNA molecule.

The present invention also provides for nucleic acid molecules having reduced secondary structure relative to a nucleic acid of substantially identical nucleotide sequence having naturally occurring bases, wherein the unstructure nucleic acid has at least two sequence elements that are substantially complementary, wherein the substantially complementary sequence elements do not form intramolecular base pairs, wherein at least one sequence element of at least two complementary sequence elements is capable of hybridizing to a substantially complementary sequence in another nucleic acid molecule.

In a preferred embodiment, unstructured nucleic acid molecules comprises nucleotides selected from the group consisting of: 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, and combinations thereof.

In another preferred embodiment, unstructured nucleic acid molecules are synthesized by an enzyme wherein the enzyme is selected from the group consisting of: an RNA polymerase, a DNA polymerase, a reverse transcriptase, a ribozyme, and a self-replicating RNA molecule.

In yet another preferred embodiment, unstructured nucleic acid molecules of the present invention are at least 40 nucleotides in length.

In yet another preferred embodiment, unstructured nucleic acid molecules of the present invention are at least 100 nucleotides in length.

In yet another preferred embodiment, unstructured nucleic acid molecules of the present invention are at least 500 nucleotides in length.

In yet another preferred embodiment, unstructured nucleic acid molecules of the present invention are used in applications including but not limited to ligase assays, polymerase extension assays, and nucleic acid arrays.

The present invention also provides for a method of producing an unstructured nucleic acid, comprising the steps of i) providing a nucleic acid template including a first nucleic acid sequence and a second nucleic acid sequence substantially complementary to said first nucleic acid sequence; ii) providing nucleic acid precursors to produce said unstructured nucleic acid which is complementary with said first and second nucleic acid sequences, said nucleotide precursors being unable to hybridise with complementary nucleotide precursors; and iii) contacting said nucleic acid template and said nucleotide precursors with an enzyme under conditions such that said unstructured nucleic acid is produced.

The present invention also provides for a method described in the preceding paragraph wherein the nucleic acid precursors comprise at least two nucleotides capable of being incorporated enzymatically into a polynucleotide, and wherein said at least two nucleotides are unable to form intramolecular base pair but can form intermolecular base pairs.

The present invention also provides for a method described in the two preceding paragraphs, wherein the sequences complementary to said first nucleic acid sequence and said one another.

The present invention also provides for a method described in the three preceding paragraphs, wherein step ii) comprises providing a) 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate; or b) 2-aminodeoxyadenosine 5'-triphosphate, 2-thiodeoxythymidine 5'-triphosphate, deoxyinosine 5'-triphosphate, deoxypyrrolopyrimidine 5'-triphosphate; or c) 2-aminodeoxyadenosine 5'-triphosphate, 2-thiodeoxythymidine 5'-triphosphate, deoxyguanosine 5'-triphosphate, 2-thiodeoxycytidine 5'-triphosphate; or d) 2-aminodeoxyadenosine 5'-triphosphate, 2-thiodeoxythymidine 5'-triphosphate, deoxyguanosine 5'-triphosphate, deoxycytidine 5'-triphosphate; or e) deoxyinosine 5'-triphosphate, deoxypyrrolopyrimidine 5'-triphosphate, deoxyadenosine 5'-triphosphate, deoxythymidine 5'-triphosphate; or f) 2-thiodeoxycytidine 5'-triphosphate, deoxyguanosine 5'-triphosphate, deoxyadenosine 5'-triphosphate, deoxythymidine 5'-triphosphate; or g) mixtures thereof.

The present invention also provides for a method described in the four preceding paragraphs, wherein said enzyme is selected from the group consisting of: RNA polymerase, DNA polymerase, reverse transcriptase, ribozymes, self-replicating RNA molecules and mixtures thereof.

The present invention also provides for an unstructured nucleic acid with reduced secondary structure relative to a nucleic acid of substantially identical nucleotide sequence having naturally occurring bases, wherein the unstructure nucleic acid has at least two sequence elements that are complementary, wherein the complementary sequence elements do not form intramolecular base pairs, wherein at least one sequence element of at least two complementary sequence element of at least two complementary sequence elements is capable of hybridizing to a substantially complementary sequence in another nucleic acid.

The present invention also provides for an unstructured nucleic acid described in the preceding paragraph, wherein the nucleic acid is synthesized by an enzyme, and optionally the enzyme is selected from the group consisting of: RNA polymerase, DNA polymerase, reverse transcriptase, ribozymes and self-replicated RNA molecules.

The present invention also provides for an unstructured nucleic acid described in the preceding two paragraphs, wherein the nucleic acid is at least 40 nucleotides in length, alternatively at least 100 nucleotides in length or alteratively at least 500 nucleotides in length.

The present invention also provides for an unstructured nucleic acid described in the preceding three paragraphs, wherein the nucleic acid comprises 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, or mixture thereof.

The present invention also provides for an unstructured nucleic acid described in any of the preceding four paragraphs producible by the methods described in any of the preceding nine paragraphs.

**Definitions**

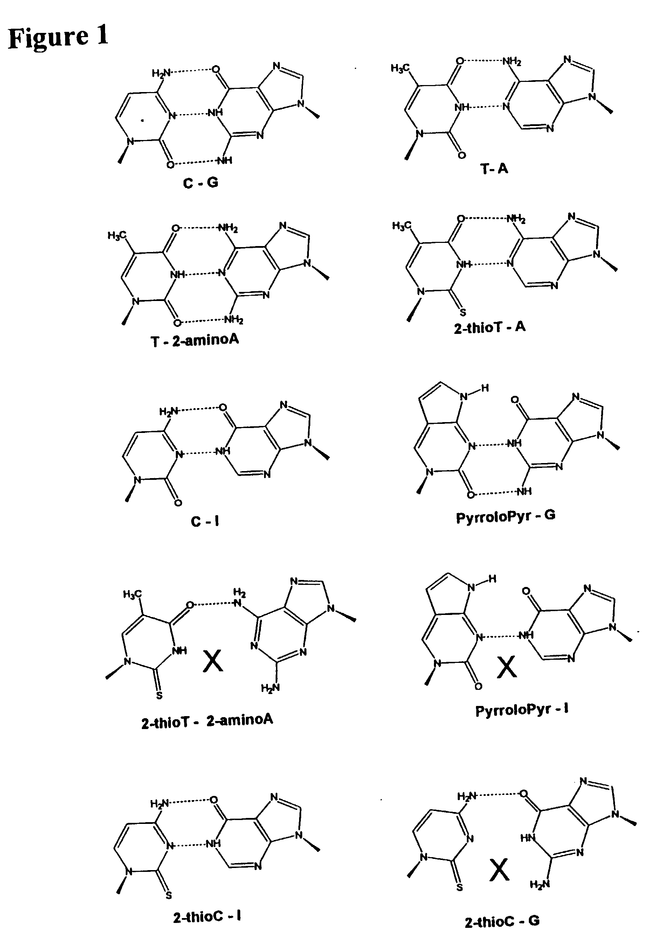

Naturally occurring bases are defined for the purposes of the present invention as adenine (A), thymine (T), guanine (G), cytosine (C), and uracil (U). The structures of A, T, G and C are shown in Figure 1. For RNA, uracil (U) replaces thymine. Uracil (structure not shown) lacks the 5-methyl group of T. It is recognized that certain modifications of these bases occur in nature. However, for the purposes of the present invention, modifications of A, T, G, C, and U that occur in nature are considered to be non-naturally occurring. For example, 2-aminoadenosine is found in nature, but is not a "naturally occurring" base as that term is used herein. Other non-limiting examples of modified bases that occur in nature but are considered to be non-naturally occurring are 5-methylcytosine, 3-methyladenine, O(6)-methylguanine, and 8-oxoguanine.

Nucleic acid bases may be defined for purposes of the present invention as nitrogenous bases derived from purine or pyrimidine. Modified bases (excluding A, T, G, C, and U) include for example, bases having a structure derived from purine or pyrimidine (i.e. base analogs). For example without limitation, a modified adenine may have a structure comprising a purine with a nitrogen atom covalently bonded to C6 of the purine ring as numbered by conventional nomenclature known in the art. In addition, it is recognized that modifications to the purine ring and/or the C6 nitrogen may also be included in a modified adenine. A modified thymine may have a structure comprising at least a pyrimidine, an oxygen atom covalently bonded to the C4 carbon, and a C5 methyl group. Again, it is recognized by those skilled in the art that modifications to the pyrimidine ring, the C4 oxygen and/or the C5 methyl group may also be included in a modified adenine. Derivatives of uracil may have a structure comprising at least a pyrimidine, an oxygen atom convalently bonded to the C4 carbon and no C5 methyl group. For example without limitation, a modified guanine may have a structure comprising at least a purine, and an oxygen atom covalently bonded to the C6 carbon. A modified cytosine has a structure comprising a pyrimidine and a nitrogen atom covalently bonded to the C4 carbon. Modifications to the purine ring and/or the C6 oxygen atom may also be included in modified guanine bases. Modifications to the pyrimidine ring and/or the C4 nitrogen atom may also be included in modified cytosine bases.

Analogs may also be derivatives of purines without restrictions to atoms covalently bonded to the C6 carbon. These analogs would be defined as purine derivatives. Analogs may also be derivatives of pyrimidines without restrictions to atoms covalently bonded to the C4 carbon. These analogs would be defined as pyrimidine derivatives. The present invention includes purine analogs having the capability of forming stable base pairs with pyrimidine analogs without limitation to analogs of A, T, G, C, and U as defined. The present invention also includes purine analogs not having the capability of forming stable base pairs with pyrimidine analogs without limitation to analogs of A, T, G, C, and U.

Complementary bases are defined according to the Watson-Crick definition for base pairing. Adenine base is complementary to thymine base and forms a stable base pair. Guanine base is complementary to cytosine base and forms a stable base pair. The base pairing scheme is depicted in Figure 1. Complementation of modified base analogs is defined according to the parent nucleotide. Complementation of modified bases does not require the ability to form stable hydrogen bonded base pairs. In other words, two modified bases may be complementary but may not form a stable base pair. Complementation of base analogs which are not considered derivatives of A, T, G, C or U is defined according to an ability to form a stable base pair with a base or base analog. For example, a particular derivative of C (i.e. 2-thiocytosine) may not form a stable base pair with G, but is still considered complementary.

In addition to purines and pyrimidines, modified bases or analogs, as those terms are used herein, include any compound that can form a hydrogen bond with one or more naturally occurring bases or with another base analog. Any compound that forms at least two hydrogen bonds with T (or U) or with a derivative of T or U is considered to be an analog of A or a modified A. Similarly, any compound that forms at least two hydrogen bonds with A or with a derivative of A is considered to be an analog of T (or U) or a modified T or U. Similarly, any compound that forms at least two hydrogen bonds with G or with a derivative of G is considered to be an analog of C or a modified C. Similarly, any compound that forms at least two hydrogen bonds with C or with a derivative of C is considered to be an analog of G or a modified G. It is recognized that under this scheme, some compounds will be considered for example to be both A analogs and G analogs.

A stable base pair is defined as two bases that can interact through the formation of at least two hydrogen bonds. Alteratively or additionally, a stable base pair may be defined as two bases that interact through at least one, preferably two, hydrogen bonds that promote base stacking interactions and therefore, promotes duplex stability.

A sequence element is defined as part or all of a polynucleotide molecule consisting of at least one nucleotide. Nucleic acid sequence is defined by the identity of the bases of nucleotides in a polynucleotide molecule.

For purposes of the present invention, two sequence elements are considered substantially complementary if at least 50% of the nucleotides in the two elements can form stable hydrogen bonds. Preferably, sequence elements are considered substantially complementary if at least 75% of the nucleotides can form stable hydrogen bonded base pairs. More preferably, sequence elements are considered substantially complementary if at least 85% of the nucleotides can form stable hydrogen bonded base pairs. Most preferably, sequence elements are considered substantially complementary if at least 95% of the nucleotides can form stable hydrogen bonded base pairs.

**Description of the Drawings**

**Figure 1.** Base-pairing schemes for natural and modified nucleotide pairs. The bold **X** indicates the disruption of the natural hydrogen-bonding interaction.

**Figure 2. A)** DNA primer and template sequence used for the polymerase extension reaction. **B)** Phosphorimage of the 10% denaturing PAGE analysis of the polymerase extension reactions. The dNTP composition (A/D, T/S, G, C) and the polymerase present in each reaction are indicated. The positions of the $^{32}$P-labeld primer and 30-mer products are indicated by arrows.

**Figure 3. A)** The 6-mer DNA primer and template sequence used to test the incorporation of the 2-amino-2'-deoxyadenosine triphosphate in a polymerase extension reaction. **B)** Phosphorimage of the 20% denaturing PAGE analysis of the polymerase extension reactions. The dATP and dDTP concentrations present in each reaction are indicated. The positions of the $^{32}$P-labeled DNA 6-mer and 7-mer products are indicated by arrows. **C)** Graphic representation of the percentage of 6-mer DNA primer converted to 7-mer DNA product as a function of dNTP concentration.

**Figure 4. A)** The 6-mer DNA primer and template sequence used to test the incorporation of the 2-thiothymidine triphosphate in a polymerase extension reaction. **B)** Phosphorimage of the 20% PAGE analysis of the polymerase extension reactions. The dTTP and 2-thioITP concentrations present in each reaction are indicated. The positions of the $^{32}$P-labeled DNA 6-mer and 7-mer products are indicated by arrows. **C)** Graphic representation of the percentage of 6-mer DNA primer converted to 7-mer product as a function of dNTP concentration.

**Figure 5. A)** The 6-mer DNA primer and template sequences used to test the incorporation of the 2-amino-2'-deoxyadenosine and 2-thiothymidine triphosphate in the polymerase extension reaction. **B)** MALDI mass spectra of the polymerase extension reactions containing the indicated dNTP. Then m/z values for the 6-mer and 7-mer extension products are indicated. **C)** Table summarizing the predicted and measured m/z values for the 6-mer and 7-mer extension products.

**Figure 6.** The scheme for generating single-stranded polynucleotides using a primer/template-dependent polymerase extension reaction followed by digestion of the template DNA with λ exonuclease.

**Figure 7.** Analysis by 10% denaturing PAGE of the single-stranded polynucleotides containing the indicated nucleotides generated according to the scheme outlined in Figure 6. The positions of size marker dyes are indicated with arrows.

**Figure 8.** Predicted secondary structures for three related 56-polynucleotide sequences containing either the four natural (A, G, C, T) nucleotides or the 2-amino-2'-deoxyadenosine (D) and 2-thiothymidine (S) nucleotide substitutions.

**Figure 9.** Ultra-violet absorption spectra of the six purified polynucleotides depicted in Figure 8.

**Figure 10.** Analysis by 10% denaturing PAGE of the six polynucleotides containing either the four natural (indicated as **A T**) nucleotides or the 2-amino-2'-deoxyadenosine (D) and 2-thiothymidine (S) nucleotide substitutions (indicated as **D S**). 0.1 and 0.2 micrograms of the templated DNA for the HP21 polymerase extension reactions was run as a standard. The size marker dyes are indicated with arrows. The gel was stained with Stains-All®.

**Figure 11.** The DNA primer and template sequences used to test the effect of the polynucleotide secondary structure on the polymerase extension reaction. The arrows indicate the direction of the polymerase extension reaction. Sequences in bold in the first three templates are derived from the primer shown in Figure 6 for the polymerization reaction used to generate each single-stranded template. -

**Figure 12.** Phosphorimages of DNA products resolved by 20% denaturing PAGE resulting from polymerase extension reactions depicted in Figure 11. The primers used, templates used, reaction products, and reaction times for each reaction are indicated.

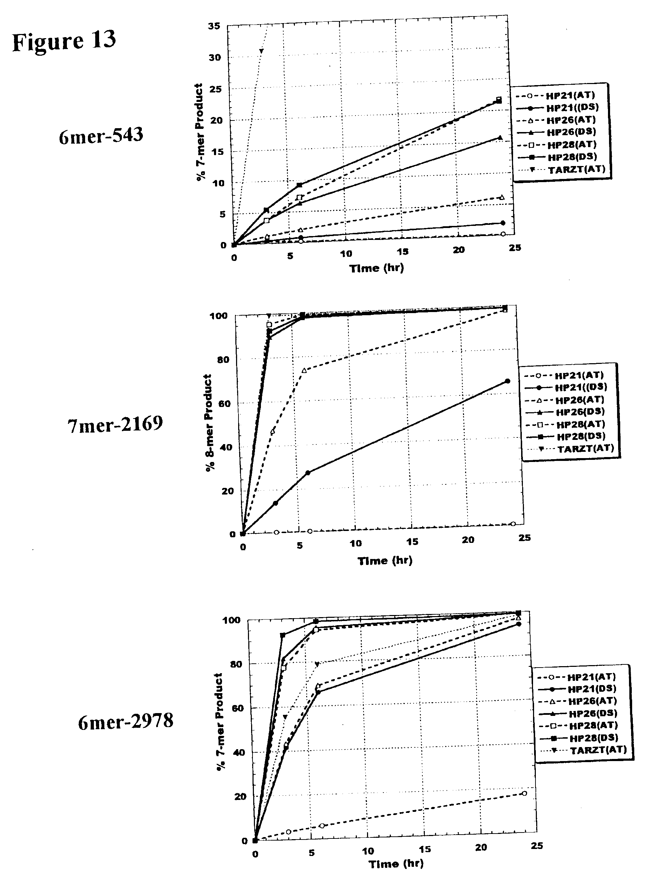

**Figure 13.** A quantitative graphic representation of the polymerase extension reactions shown in Figure 12. The graphs indicate the percentage of primers converted to the reaction products.

## Description of Certain Preferred Embodiments

The present invention provides a system for generating nucleic acid molecules having reduced levels of secondary structure compared to nucleic acid molecules of the same nucleotide sequence containing only naturally-occurring bases. Such molecules are referred to herein as "unstructured nucleic acids" (UNAs). UNAs have reduced levels of secondary structure because of their reduced ability to form intramolecular hydrogen bond base pairs between regions of substantially complementary sequence. Preferred UNAs, however, retain the ability to form intermolecular hydrogen bond base pairs with other nucleic acid molecules.

UNAs contain nucleotide base analogs or a mixture of base analogs and naturally-occurring bases such that regions of sequence complementarity within the UNA are unable to form base pairs. One or both of the nucleotides that together form an intramolecular complementary base pair are substituted with a nucleotide containing a base analog so that the base pair is no longer formed, or is only formed at a reduced level. Preferably, the reduced level of base pairing is no more than one hydrogen bond interaction. Preferably, the analog(s) is selected so that the UNAs retain the ability to hybridize with another nucleic acid molecule of complementary or substantially complementary sequence.

The base pairing concepts of the present invention are schematically depicted by the following formulas where A' ≠ T' and G' ≠ C' represent disallowed base-pairing schemes, with the symbol ≠ representing the inability to form a base pair. [A*, T*, G*, and C*] represent a second group of bases capable of forming base pairs with A', T', G' and C' according to the general Watson-Crick base pair scheme of A=T and G=C , where = represents the ability to form a base pair. The same base pairing rules apply for RNA where U replaces T. (The horizontal base pairing symbols are not meant to represent the number of hydrogen bonds present in the base pair, but are meant only to indicate a stable base pair or lack of a stable base pair.)

$$A' \neq T'; \; G' \neq C' \qquad (1)$$

$$(A'=T^*; \; T'=A^*; \; G'=C^*; \; C'=G^*) \qquad (2)$$

Formula 1 indicates that base pair analogs A'/T' and G'/C' are unable to form a stable base pair. However, as indicated in Formula 2, the bases of nucleotides A' T' G' and C' are capable of forming stable base pairs with a second group of nucleotide bases (A* T* G* C*).

UNAs of the present invention may contain a mixture of nucleotide analogs and naturally-occurring nucleotides. UNAs of the present invention may also contain only nucleotide base analogs. More specifically, in accordance with the base pairing formulas outlined in Formula 1 and 2, nucleotides of the first group (A', T', G', C') and nucleotides of the second group (A*, T*, G*, and C*) may include combinations of natural bases and modified bases or include all modified bases. For example, A' and T', which does not form a stable base pair, may be comprised of one nucleotide base analog (A') and one natural nucleotide (T'). Alternatively, A' and T' may be comprised of two nucleotide base analogs. Nucleotide pairs from the second group (e.g. A* and T*) may or may not form stable base pairs ( A*=T* or A*≠T*).

UNAs of the present invention may contain both A'/T' base pair analogs that do not form stable base pairs and G/C base pairs that do form stable base pairs. Alternatively, UNAs may contain G'/C' base pair analogs that do not form stable base pairs and A/T base pairs that do form stable base pairs. UNAs of the present invention may also contain both sets of analogs that do not form stable base pairs (A' ≠ T' and G' ≠ C'). For the present invention, nucleotide from the first and second class (e.g. A', A*) may be mixed in the same molecule. However, it is preferred that a single UNA molecule possess no more than one of each type of nucleotide (e.g. only A' T' G and C) which results in only one type of base-pairing scheme for each potential base-pair.

## Methods of Producing UNAs

It is an aspect of the present invention that polynucleotides with reduced levels of intramolecular base pairing (secondary structure) and preferably also with the ability to hybridize to other nucleotide molecules of substantially complementary sequence (intermolecular base pairing) are produced by any method which incorporates nucleotides having naturally occurring and/or modified bases. Preferably, UNAs according to the present invention are produced enzymatically.

Oligonucleotides and polynucleotides containing nucleotide base, ribose and phosphate backbone modifications have been chemically synthesized using methods known in the art (Beaucage and Caruthers, *Tetrahedron Letters*, 22; 1859-1862 (1981)). Current limitations in the chemistry of the nucleic acid synthesis, such as yield and purity, restrict the size of oligonucleotides synthesized chemically to approximately 100 nucleotides in length. However, the present invention does not preclude the chemical synthesis of UNAs greater than 100 nucleotides in length.

In a preferred embodiment, UNAs of the present invention are produced enzymatically. Polymerization methodologies that utilize template dependent DNA or RNA polymerases are preferred methods for copying genetic material of unknown sequence from biological sources for subsequent sequence and expression analyses. However, it is recognized that preferred UNAs of the present invention may be produced by any method and is not limited to enzymatic methods. Thus UNAs, which are produced preferably by enzymatic methods, are well suited for generating oligonucleotides and polynucleotides for subsequent genetic and expression analysis. Moreover, since preferred UNAs of the present invention are generated using DNA and RNA polymerases, the invention is able to generate oligonucleotides and polynucleotides anywhere from 8 to several thousand nucleotides in length. Preferably, UNA's of the present invention are at least 40 nucleotides in length. More preferably, UNA's of the present invention are at least 100 nucleotides in length. Most preferably, UNA's of the present invention are at least 500 nucleotides in length.

Any enzyme capable of incorporating naturally-occurring nucleotides, nucleotides base analogs, or combinations thereof into a polynucleotide may be utilized in accordance with the present invention. As examples without limitation, the enzyme can be a primer/DNA template dependent DNA polymerase, a primer/RNA template dependent reverse transcriptase or a promoter-dependent RNA polymerase. Non-limiting examples of DNA polymerases include *E. coli* DNA polymerase I, *E. coli* DNA polymerase I Large Fragment (Klenow fragment), or phage T7 DNA polymerase. The polymerase can be a thermophilic polymerase such as *Thermus aquaticus* (Taq) DNA polymerase, *Thermus flavus* (Tfl) DNA polymerase, *Thermus Thermophilus* (Tth) Dna polymerase, *Thermococcus litoralis* (Tli) DNA polymerase, *Pyrococcus furiosus* (Pfu) DNA polymerase, Vent™ DNA polymerase, or *Bacillus stearothermophilus* (Bst) DNA polymerase. Non-limiting examples of reverse transcriptases include AMV Reverse Transcriptase, MMLV Reverse Transciptase and HIV-1 reverse transcriptase. Non-limiting examples of RNA polymerases suitable for generating RNA version of UNAs include the bacteriophage RNA polymerases from SP6, T7 and T3. Furthermore, any molecule capable of using a DNA or an RNA molecule as a template to synthesize another DNA or RNA molecule can be used in accordance with the present invention. (e.g. self-replicating RNA).

Primer/DNA template-dependent DNA polymerases, primer/RNA template-dependent reverse transcriptases and promoter-dependent RNA polymerases incorporate nucleotide triphosphates into the growing polynucleotide chain according to the standard Watson and Crick base-pairing interactions (see for example; Johnson, Annual Review in *Biochemistry*, 62; 685-7 13 (1993), Goodman et al., Critical Review in *Biochemistry* and Molecular Biology, 28; 83-126 (1993) and Chamberlin and Ryan, The Enzymes, *ed.* Boyer, Academic Press, New York, (1982) pp 87-108). Some primer/DNA template dependent DNA polymerases and primer/RNA template dependent reverse transcriptases are capable of incorporating non-naturally occurring triphosphates into polynucleotide chains when the correct complementary nucleotide is present in the template sequence. For example, Klenow fragment and AMV reverse transcriptase are capable of incorporating the base analogue iso-guanosine opposite iso-cytidine residues in the template sequence (Switzer et al., *Biochemistry* 32; 10489-10496 (1993). Similarly, Klenow fragment and HIV-1 reverse transcriptase are capable of incorporating the base analogue 2,4-diaminopyrimidine opposite xanthosine in a template sequence (Lutz et al., *Nucleic Acids Research* 24; 1308-1313 (1996)).

UNAs may also be generated using one of a number of different methods known in the art. These include but are not limited to nick translation for generating labeled target molecules (Feinberg and Vogelstein, *Analytical Biochemistry*, 132; 6-13 (1983) and Feinberg and Vogelstein, *Analytical Biochemistry*, 137; 266-267 (1984)), asymmetric PCR methods (Gyllensten and Erlich, *Proc. Natl. Acad. Sci.* USA. 85; 7652-7656(1988)) that utilize a single primer or a primer having some chemical modification that results in the synthesis of strands of unequal lengths (Williams and Bartel, *Nucleic Acids Research*, 23; 4220-4221 (1995) and affinity purification methods that utilize either magnetic beads (Hultman et al., *Nucleic Acids Research*, 17; 4937-4946 (1989)) or streptavidin induced electrophoretic mobility shifts (Nikos, *Nucleic Acids Research*, 24; 3645-3646 (1996)).

The asymmetric PCR method would be performed using a single target-specific primer and either a single-stranded or double stranded DNA template in the presence of a thermophylic DNA polymerse or reverse transcriptase and the appropriate UNA nucleotide triphosphates. The reaction mixture would be subjected to temperature cycle a defined number of times depending upon the degree of amplification desired. The limitation of the amplification to this type of linear mode is inherent to the designed base-pairing properties of UNAs. Unlike nucleic acids generated from the four standard nucleotides, the UNA replication products are generated from non-complementary pairs nucleotides and thus cannot serve as templates for subsequent replication events. However the invention does not preclude the use of PCR to amplify the target prior to generation of UNAs by the invention.

UNAs can also be generated using a polymerase extension reaction followed by a strand-selective exonuclease digestion (Little et al., J. Biol Chem. 242, 672 (1967) and Higuchi and Ochamn, *Nucleic Acids Research*, 17; 5865- (1989)). For example, a target-specific primer is extended in an isothermal reaction using a DNA polymerase or reverse transcriptase in the presence of the appropriate UNA nucleotide triphosphates and a 5'-phosphorylated DNA template. The DNA template strand of the resulting duplex is then specifically degraded using the 5'-phosphorly-specific lambda exonuclease. A kit for performing the latter step is the Strandase Kit™ currently marketed by Novagen (Madison, WI).

Single-stranded ribonucleotide (RNA) versions of UNAs can be synthesized using *in vitro* transcription methods which utilize phage promoter-specific RNA polymerases such as SP6 RNA polymerase, T7 RNA polymerase and T3 RNA polymerase (see for example Chamberlin and Ryan, The Enzymes, *ed.* Boyer, Qacademic Press, New York, (1982) pp87-108 and Melton et al., *Nucleic Acids Research*, 12; 7035 (1984)). For these methods, a double stranded DNA corresponding to the target sequence is generated using PCR methods known in the art in which a phage promoter sequence is incorporated upstream of the target sequence. This double-stranded DNA is then used as the template in an *in vitro* transcription reaction containing the appropriate phage polymerase and the ribonucleotide triphosphate UNA analogues. Alternatively, a single stranded DNA template prepared according to the method of Milligan and Uhlenbeck, (Methods in Enzymology, 180A, 51-62 (1989)) can be used to generate RNA versions of UNAs having any sequence. A benefit of these types of *in vitro* transcription methods is that they can result in a 100 to 500 fold amplification of the template sequence.

## Structural Modifications to Nucleotides

Nucleotide base analogues having fewer structural changes can also be efficient substrates for DNA polymerase reactions. For example, a number of polymerases can specifically incorporate inosine across cytidine residues (Mizusawa et al., *Nucleic Acids Research*, 14; 1319 (1986). The analogue 2-aminoadenosine triphosphate can also be efficiently incorporated by a number of DNA polymerases and reverse transcriptases (Bailly and Waring, Nucleic Acids Research, 23; 885 (1996). In fact, 2-aminoadenosine is a natural substitute for adenosine in S-2L cyanophage genomic DNA. However, for the present invention 2-aminoadenosine is defined as a non-naturally occurring base. The 2-aminoadenosine ribonucleotide-5'-triphosphate is a good substrate *for E. coli* RNA polymerase (Rackwitz and Scheit, Eur. J. Biochem., 72, 191 (1977)). The adenosine analogue 2-aminopurine can also be efficiently incorporated opposite T residues by *E. coli* DNA polymerase (Bloom et al., *Biochemistry* 32; 11247-11258 (1993) but can mispair with cytidine residues as well (see Law et al., Biochemsitry 35; 12329-12337 (1996)).

Any structural modifications to a nucleotide that do not inhibit the ability of an enzyme to incorporate the nucleotide analogue may be used in the present invention if the modifications do not result in a violation of the base pairing rules set forth in the present invention. Modifications include but are not limited to structural changes to the base moiety (e.g. C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine), changes to the ribose ring (e.g. 2'-hydroxyl, 2'-fluro), and changes to the phosphodiester linkage (e.g. phosphorothioates and 5' -N-phosphoamidite linkages).

Watson-Crick base-pairing schemes can accommodate a number of modifications to the ribose ring, the phosphate backbone and the nucleotide bases (Saenger, Principles of Nucleic Acid Structure, Springer-Verlag, New York, NY. 1983). Certain modified bases such as inosine, 7-deazaadenosine, 7-deazaguanosine and deoxyuridine decrease the stability of base-pairing interactions when incorporated into polynucleotides. The dNTP forms of these modified nucleotides are efficient substrates for DNA polymerases and have been used to reduce sequencing artifacts that result from target and extension product secondary structures (Mizusawa et al., *Nucleic Acids Research*, 14; 1319. 1986). Other modified nucleotides, such as 5-methylcytidine, C-5 propynyl-cytidine, C-5 propynyl-uridine and 2-aminoadenosine increase the stability of duplex when incorporated into polynucleotides (Wagner et al., *Science*, 260; 1510. 1993) and have been used to increase the hybridization efficiency between oligonucleotide probes and target sequences.

## Selection of Nucleotides for UNAs

In accordance with the present invention, UNAs are produced such that sequence elements in the UNA have a reduced ability to hybridize to substantially complementary sequence elements within the same UNA polynucleotide molecule. Complementary nucleotides for producing UNAs are selected such that a first nucleotide base is not capable of forming a stable base pair with a nucleotide complement. The two complementary nucleotides may have one naturally-occurring base and one base analog or may have two base analogs. The complementary nucleotides that are unable to form a stable base pair are used to produce UNA with reduce the levels of intramolecular base pairing by reducing hybridization between sequence elements within the UNA that are substantially complementary. Complementary nucleotides that are unable to form stable pairs may also be used in sequences of the UNA that do not have substantially self-complementary sequences within the same UNA polynucleotide molecule.

In addition, it is preferable that the complementary nucleotides in a UNA that are unable to form stable base pairs, are capable of forming stable base pairs with at least one nucleotide complement present in a second polynucleotide molecule. Preferably, the second polynucleotide molecule contains sequences elements substantially complementary to sequence elements in the UNA to allow hybridization of part or all of the second polynucleotide to the UNA. Complementary sequence elements of the second polynucleotide may contain naturally-occurring bases or base analogs.

## 2-Aminoadenosine (D), 2-Thiothymidine (2-thioT), Inosine (I) and Pyrrolo-pyrimidine (P)

In another preferred embodiment of the present invention, the nucleotide analogs 2-aminoadenosine (D), 2-thiothymidine (2-thioT), inosine (I) and pyrrolo-pyrimidine (P) are used to generate nucleic acid molecules that are unable to form stable secondary structures yet retain their ability to form Watson-Crick base-pairs with oligonucleotides composed of the four natural bases. The structures of the D/2-thioT, I/P and the four natural base pairs along with various combinations of the natural and base analogs are shown in Figure 1.

Naturally occurring Watson-Crick base-pairing is defined by specific hydrogen bonding interactions between the bases of adenine and thymine (or uracil) and between guanine and cytosine. Positioning of hydrogen-bond donors (e.g. amino groups) and hydrogen-bond acceptors (e.g. carbonyl groups) on purine and pyrimidine bases place structural constraints on the ability of two nucleoside bases to form stable hydrogen bonds. Figure 1 shows the structures of the bases and the relative orientations of the bases to each other in a Watson-Crick base pair. In addition, an inosine:cytosine base pair is shown. The inosine-cytosine base pair is identical to a G-C base pair except that the I-C base pair lacks the hydrogen bond donor of the 2-amino group of guanine which is missing in inosine.

## 2-Aminoadenosine (D), 2-Thiothymidine (2-thioT)

Without being limited by theory, a D/2-thioT base pair analog is prevented from forming a stable base pair presumably due to a steric clash between the thio group of 2-thioT and the exocyclic amino group of 2-aminoadenosine as a result of the larger atomic radius of the sulfur atom. This tilts the nucleotide bases relative to one another such that only one hydrogen bond is able to form. It is also known that thionyl sulfur atoms are poorer hydrogen-bonding acceptors than carbonyl oxygen atoms which could also contribute to the weakening of the D/2-thioT base pair.

Furthermore, the 2-aminoadenosine (D) is capable of forming a stable base-pair with thymidine (T) through three hydrogen bonds in which a third hydrogen bonding interaction is formed between the 2-amino group and the C2 carbonyl group of thymine. As a result, the D/T base pair is more stable thermodynamically than an A/T base pair. In addition, 2-thiothymidine (2-thioT) is capable of forming a stable hydrogen bonded base pair with adenosine (A) which lacks an exocyclic C2 group to clash with the 2-thio group.

Therefore, polynucleotide molecules with 2-aminoadenosine (D) and 2-thioT replacing A and T respectively are unable to form intramolecular D/2-thioT base pairs but are still capable of hybridizing to polynucleotides of substantially complementary sequence comprising A and T and lacking D and 2-thioT. Without being limited by theory, the aforementioned proposed mechanisms regarding the factors responsible for stabilizing and disrupting the A/T and G/C analogue pairs are not meant in anyway to limit the scope of the present invention and are valid irrespective of the nature of the specific mechanisms.

Gamper and coworkers (Kutyavin et al. *Biochemistry*, 35; 11170 (1996)) determined experimentally that short oligonucleotide duplexes containing D/T base pairs that replace A/T base pairs have melting temperatures (Tm) as much as 10° C higher than duplexes of identical sequence composed of the four natural nucleotides. This is due mainly to the extra hydrogen bond provide by the 2-amino group. However, the duplexes designed to form opposing D/2-thioT base-pairs exhibited Tms as much as 25° C lower than the duplex of identical sequence composed of standard A/T base-pairs. The authors speculate that this is mainly due to the steric clash between the 2-thio group and the 2-amino group which destabilizes the duplex. Deoxyribonucleotides in this study were synthesized using chemical methods.

Although the base-pairing selectivity for these analog pairs has been experimentally tested for only DNA duplexes, it is likely that these same rules will hold for RNA duplexes and DNA/RNA heteroduplexes as well. This would allow for RNA versions of UNAs to be generated by transcription of PCR or cDNA products using the ribonucleotide triphosphate forms of the UNA analog pairs and RNA polymerases.

## Inosine (I) and Pyrrolo-pyrimidine (P)

The inosine (I) and pyrrolo-pyrimidine (P) I/P base pair analog is also depicted in Figure 1. Inosine, which lacks the exocyclic 2-amino group of guanine, forms a stable base pair with cytosine through two hydrogen bonds (vs. three for G/C). The other member of the I/P analog is pyrrolo-pyrimidine (P) which is capable of forming a stable base pair with guanine despite the loss of the 4-amino hydrogen bond donor of cytosine. Figure 1 shows that a P/G base pair is also formed through two hydrogen bonds. The N7 group of P is spatially confined by the pyrrole ring and is unable to form a hydrogen bond with the C6 carbonyl O of guanine. However, this does not prevent the formation of the other two hydrogen bonds between P/G. The I/P base pair is only capable of forming one hydrogen bond (as depicted in Figure 1) and is therefore not a stable base pair. As a result, polynucleotide molecules with I and P replacing G and C respectively are unable to form intramolecular I/P base pairs but are still capable of hybridizing to polynucleotides of substantially complementary sequence comprising G and C and lacking I and P.

Woo and co-workers (Woo et al., *Nucleic Acids Research*, 24; 2470 (1996)) showed that introducing either P or I into 28-mer duplexes to form P/G and I/C base-pairs decreased the Tm of the duplex by -0.5 and -1.9° C respectively per modified base-pair. These values reflect the slight destabilization attributable to the G/P pair and a larger destabilization due to the I/C pair. However, introducing P and I into the duplexes such that opposing I/P base-pairs are formed reduced the Tm by -3.3° C per modified base-pair. Therefore the I/P base pairs are more destabilizing.

## UNAs comprising D, 2-thioT, I, and P

In accordance with the present invention, nucleic acid molecules with reduced secondary structure (UNAs) are generated by performing primer dependent, template directed polymerase reactions using the nucleotide 5'-triphosphate forms of the appropriate analog pairs. These include; 2-amino-2'-deoxyadenosine-5'-triphosphate (dDTP), 2-thiothymidine-5'-triphosphate (2-thioTTP), 2'-deoxyinosine-5'-triphosphate (dITP) and 2'-deoxypyrrolo-pyrimidine-5'-triphosphate (dPTP). For example, a reaction containing dDTP, 2-thioTTP, dCTP and dGTP will generate UNAs which are unable to form intramolecular A/T base pairs. Likewise, a reaction containing dATP, dTTP, dPTP and dITP will generate UNAs which are unable to form intramolecular P/I (modification of G/C) base pairs. A polymerization reaction containing both analog pairs, dDTP, 2-thioTTP; and dPTP, dITP will generate UNAs that have no predicted intramolecular base-pairing interactions. However, since 2-aminoadenosine, 2-thiothymidine, pyrrolo-pyrimindine, and inosine are still capable of forming stable base pairs with thymidine, adenosine, cytidine and guanosine respectively, all three types of UNAs should be able to specifically hybridize intermolecularly to oligonucleotides composed of the four natural bases.

In yet another preferred embodiment, it is recognized that UNAs of the present invention may contain various levels of secondary structure. For example, UNAs may contain only G/C intramolecular base pairs and not A/T intramolecular base pairs. Alternatively, UNAs may contain only A/T intramolecular base pairs and not G/C intramolecular base pairs. As described in Examples 1 and 2 but without limitations to only those experimental conditions, UNAs potentially containing only G/C intramolecular base pairs are generated by enzymatically incorporating the triphosphate forms of 2-aminoadenosine, 2-thiothymidine, guanosine, and cytosine into a polynucleotide. The resulting UNA polynucleotide is not capable of forming intramolecular A/T base pairs, but is still capable of forming intramolecular G/C base pairs. The aforementioned mechanisms which may account for the observed disruption of the A/T and G/C analogue pairs is not meant in anyway to limit the scope of the present invention and is valid irrespective of the nature of the specific mechanisms.

## UNAs comprising D, 2-thioT, 2-thioC, and G

In yet another preferred embodiment of the present invention, the nucleotide base pair analogs D/2-thiothymidine and 2-thiocytosine/guanosine (2-thioC/G) are used in primer dependent polymerase reactions to generate nucleic acid molecules that are unable to form stable secondary structures yet retain their ability to form Watson-Crick base pairs with oligonucleotides composed of the four natural bases. 2-thioC and G are unable to form a stable base pair. The presence of a 2-thiol exocyclic group in cytosine replacing the C2 carbonyl group effectively removes the hydrogen bond acceptor at that position and causes a steric clash due to the large ionic radius of sulfur as compared to oxygen. As a result, 2-thioC/G is only capable of forming a single hydrogen bond and is thus not a stable base pair. However, 2-thioC and I are capable of forming a stable base pair through two hydrogen bonds since the removal of the 2-amino exocyclic group of guanine that results in inosine effectively removes the steric clash between the C2 sulfur of 2-thioC and the 2-amino group of guanine.

Therefore, polynucleotide molecules with reduced secondary structure are generated enzymatically using the 5'-triphosphate forms of the base pair analogs. These include; 2-amino-2'-deoxyadenosine-5'-triphosphate (dDTP), 2-thiothymidine-5'-triphosphate(2-thioTTP), 2'-deoxyguanosine-5'-triphosphate (dGTP) and 2-thio-2'-deoxycytidine-5'-triphosphate (2-thio-dCTP). For example, a reaction with 2-thio-dCTP, dGTP, dATP, dTTP will generate UNA=s that can form only A/F base pairs. A polymerization reaction containing both analog pairs, 2-thio-dCTP/dGTP, and dDTP/2-thioTTP will generate UNAs that have no predicted intramolecular base-pairing interactions. However, since 2-aminoadenosine, 2-thiothymidine, 2-thiocytidine and guanosine are still capable of forming stable base pairs with thymidine, adenosine, inosine and cytidine respectively, UNAs comprising (A, T, 2-thioC, G) or (D, 2-thioT, 2-thioC, G) should be able to specifically hybridize to oligonucleotides composed of the appropriate bases according to the base pairing rules discussed.

The 2-thioC/G base pair analog provides an example of a base pair analog comprising a natural nucleotide base and a nucleotide base analog which can not form a stable base pair. As previously stated, polynucleotides containing 2-thiocytidine and guanosine cannot form intramolecular 2-thioC/G base pairs. However, these polynucleotides can form base pairs with polynucleotides of substantially complementary sequences through 2-thioC/I and C/G base pairs. Therefore, UNAs comprising 2-thioC/G are capable of hybridizing to polynucleotide molecules also containing base analogs (inosine).

## Methods of Utilizing UNAs

The ability to generate nucleic acids that retain their genetic information content yet possess little or no secondary structure has many advantages. Methods used in molecular biology and nucleic acids chemistry that rely on the hybridization of single-stranded nucleic acid probes to single-stranded nucleic acid targets of substantially complementary sequence can utilize UNAs in accordance with the present invention (for general protocols, see Ausubel *et al. Current Protocols in Molecular Biology*. John Wiley & Sons, Inc. 1998; Sambrook *et al., Molecular Cloning: A Laboratory Manual*, Cold Spring Harbor Laboratory Press, Cold Spring Harbor, NY, 1989; both of which are incorporated herein by reference). By way of examples and not limitations to the present invention, these methods include methods of in situ hybridization (*e.g.* mRNA fluorescence in situ hybridization; FISH), surface-bound oligonucleotide arrays for mutation detection (*e.g.* gene chip technology, Chee, M., et al., (1996) *Science* 274, 610-614, Wang, et. al., *Science*, 280, 1077-1082), gene expression assays (Lockart, D., et al., (1996) *Nat. Biotech.* 14, 1675-1680), the polymerase chain reaction (PCR), RNA (e.g. Northern blot) and DNA (e.g. Southern blot) blot hybridization, DNA sequencing including high-throughput, primer extension analysis, screening recombinant DNA libraries, polymerase extension assays, and X-mer ligase assays.

Techniques employing ligase assays and polymerase extension assays are useful for determining whether a mutation is present at a defined location in an otherwise known target nucleic acid sequence (see for example; Haff and Smirnov, *Genome Research*, 7; 378-388 (1997), Landegren et al., *Genome Research*, 8; 769-776 (1998), Shumaker et al., *Human Mutation*, 7; 346-354 (1996), Pastinen et al., *Genome Research*, 7; 606-6 14 (1997), and Nikiforov et al., *Nucleic Acids Research*, 22; 4167-4175 (1994)). U.S. Patent No. 4,988,617, for example, discloses a method for determining whether a mutation is present at a defined location in an otherwise known target nucleic acid sequence by assaying for the ligation of two natural oligonucleotides that are designed to hybridize adjacent to one another along the target sequence. U.S. Patent No. 5,494,810 discloses a method that utilizes a thermostable ligase and the ligase chain reaction (LCR) to detect specific nucleotide substitutions, deletions, insertions and translocations within an otherwise known target nucleic acid sequence using only natural nucleic acids. U.S. Patent No. 5,403,709 discloses a method for determining the nucleotide sequence by using another oligonucleotide as an extension and a third, bridging oligonucleotide to hold the first two together for ligation, and WO 97/35033 discloses methods for determining the identity of a nucleotide 3' to a defined primer using a polymerase extension assay.

U.S. Patent Nos. 5,521,065, 4,883,750 and 5,242,794 disclose methods of testing for the presence or absence of a target sequence in a mixture of single-stranded nucleic acid fragments. The method involves reacting a mixture of single-stranded nucleic acid fragments with a first probe that is complementary to a first region of the target sequence and with a second probe that is complementary to a second region of the target sequence. The first and second target regions are contiguous with one another. Hybridization conditions are used in which the two probes become stably hybridized to their associated target regions. Following hybridization, any of the first and second probes hybridized to contiguous first and second target regions are ligated, and the sample is subsequently tested for the presence of expected probe ligation product.

US Patent application 09/112437 discloses a generic methods and reagents for analyzing the nucleotide sequence of nucleic acids using high-throughput mass spectrometry (incorporated herein by reference in its entirety). The disclosed methods and reagents utilize complex mixtures of short (e.g. 6-mer and 7-mer) oligonucleotides (X-mers) and polymerase extension and ligation reactions to generate a complex mixture of oligonucleotide products that reflect the sample's DNA sequence. Because the methods rely on hybridization between the short X-mers and the target polynucleotide, UNAs would serve as superior targets for both the polymerase extension assay (PEA) and X-mer ligase assays (XLA). This is because the X-mers would not have to compete with intramolecular target sequences for their complementary binding site. Minimizing the effect of target structure makes the problem of equalizing the hybridization rates and subsequent polymerase extension reaction rates for each X-mer more straight-forward.

## Array Technology

The present invention would also greatly benefit gene expression assays. The current art requires considerable probe design and probe redundancy to ensure that the probes are targeted to regions of the mRNA species (or cDNA) which are not involved in the intramolecular structures and therefor capable of hybridizing with the probes. Employing UNAs as the target would minimize if not eliminate the need for this design aspect and would likely increase the overall sensitivity of the gene expression assays. Importantly, UNAs would greatly facilitate the use of short oligonucleotide arrays for mutation scanning and detection since these applications, by definition, require that all regions of the target sequence be accessible for hybridization by the interrogating probes.

Techniques employing hybridization to surface-bound DNA probe arrays are useful for analyzing the nucleotide sequence of target nucleic acids. These techniques rely upon the inherent ability of nucleic acids to form duplexes via hydrogen bonding according to Watson-Crick base-pairing rules. In theory, and to some extent in practice, hybridization to surface-bound DNA probe arrays can provide a relatively large amount of information in a single experiment. For example, array technology has identified single nucleotide polymorphisms within relatively long (1,000 residues or bases) sequences (Kozal, M., *et al., Nature Med.* 7:753-759, July 1996). In addition, array technology is useful for some types of gene expression analysis, relying upon a comparative analysis of complex mixtures of mRNA target (Lockart, D., et al., *Nat. Biotech.* 14, 1675-1680. 1996). Although array technologies offer the advantages to being reasonably sensitive and accurate when developed for specific applications and for specific sets of target sequences, they lack a generic implementation that can simultaneously be applied to multiple and/or different applications and targets. This is in large part due to the need for relatively long probe/target duplexes. Moreover, this use of relatively long probes makes it difficult to interrogate single nucleotide differences due to the inherently small thermodynamic difference between the perfect complement and the single mismatch within the probe/target duplex. In addition, detection depends upon solution diffusion properties and hydrogen bonding between complementary target and probe sequences. Therefore, utilizing UNAs of the present invention as nucleic acid targets will enable the use short oligonucleotide probes for differentiating between nucleic acid molecules differing in sequence by a few or even a single nucleotide.

It is appreciated by those of ordinary skill in the art that other modified nucleotides, nucleic acid polymerization enzymes and methods for generating UNAs not described in the specification may be used in accordance with the present invention. The following Examples are meant to provide experimental detail to the present invention and are not meant to limit the scope of the present invention.

**Examples**

**Example 1: Materials**

**Preparation of the 2-amino-2'-deoxyadenosine-5'-triphosphate and 2-thiothymidine-5'-triphosphate**

The 2-amino-2'-deoxyadenosine and 2-thiothymidine nucleosides were purchased from Chemgenes (Waltham MA) and Berry & Associates (Dexter MI) respectively. The nucleoside 5'-triphosphates were prepared by phosphorylation of the unprotected nucleosides using anhydrous phosphoryl chloride ($POCl_3$) and purified by chromatography on DEAE-sephadex according to the method of Seela and Gumbiowski (Helvetica Chimica Acta, (1991) 74, 1048). The purified nucleoside 5-triphosphates were >95% pure as determined by $^{31}P$ NMR and HPLC. The extinction coefficients ($mol^{-1}$ $cm^{-1}$) for the 2-amino-2'-deoxyadenosine-5'-triphosphate (dDTP) and 2-thiothymidine-5-triphosphate (2-thioTTP) are $lambda_{255}$= 7,600 and $lambda_{276}$=16,600 respectively.

**Example 2: Incorporation of the 2-amino-2'-deoxyadenosine-5'-triphosphate and 2-thiothymidine-5-triphosphate into Polynucleotides by DNA Polymerases**

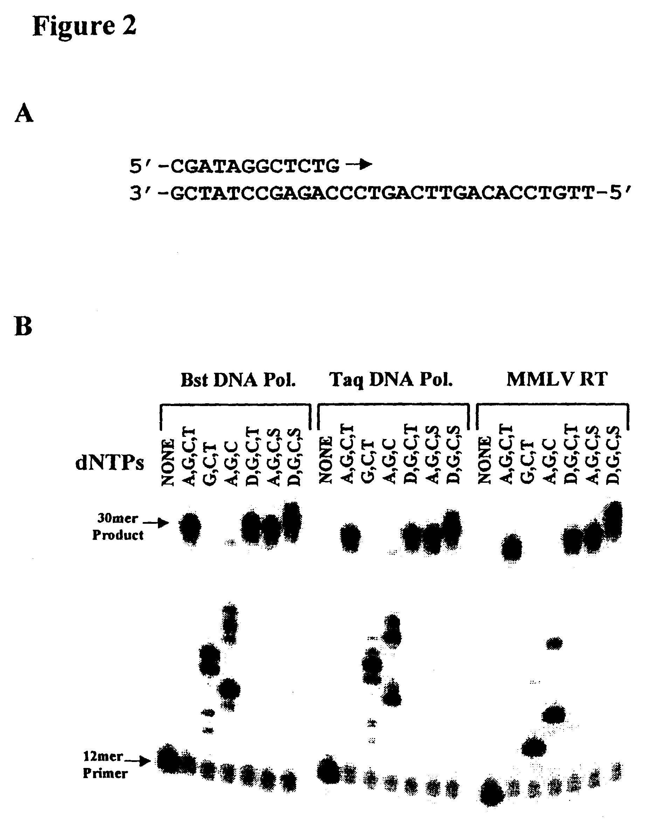

The ability of the *Bacillus sterothermophilus* (Bst) DNA polymerase (New England Biolabs), the *Thermus aquaticus* (Taq) DNA polymerase (Amersham) and the Moloney Murine Leukemia Virus reverse transcriptase (MMLV-RT) (Amersham) to incorporate the dDTP and 2-thioTTP into polynucleotides was tested using a synthetic 30-mer template and $^{32}P$-labeled 12-mer primer (S.A. 130 Ci/mmol). (Figure 2A). Extension reactions for each polymerase were performed in 0.65ml pre-siliconized microfuge tubes containing the following components: **(Bst)** 20 mM Tris-Cl (pH 8.8 @ 25° C), 10 mM KCl, 10 mM $(NH_4)_2SO_4$, 2 mM $MgSO_4$, 0.1% Triton-X100, 0.5 microM primer/template, 250 microM each dNTP and 0.15 units/microL Bst DNA polymerase; **(Taq)** 26 mM Tris-Cl (pH 9.5 @25° C), 6.5 mM $MgCl_2$, 0.5 microM primer/template, 250 microM each dNTP and 0.15 units/microL Taq DNA polymerase; **(MMLV-RT)** 50 mM Tris-Cl (pH 8.3 @ 25° C) 75 mM KCl, 3 mM $MgCl_2$, 1 M DTT, 0.5 microM primer/template, 250 microM each dNTP and 0.5 units/microL MMLV-RT. The reactions were incubated for 15 minutes at 65° C for the Bst and Taq reactions and 42° C for the MMLV-RT reaction. The reaction mixtures were separated by electrophoresis on 10% denaturing (7M urea) polyacrylamide gels and visualized by phosphorimaging methods.

In the reaction mixtures containing only dGTP, dCTP, dTTP or dATP, dGTP and dCTP, no full-length product was generated by any of the three polymerases (Figure 2B). However, when all four dNTPs were present or when dDTP (D) was substituted for dATP or 2-thioTTP (S) was substituted for dTTP, greater than 90% of the primer was converted to full-length 30-mer product. The incorporation of the dDTP by the Taq DNA polymerase is consistent with the results of Bailly and Waring (*Nucleic Acids Research*, 23:885. 1995). Importantly, full-length product was generated by all three polymerases when both the D and S are substituted for A and T in a single reaction mixture.

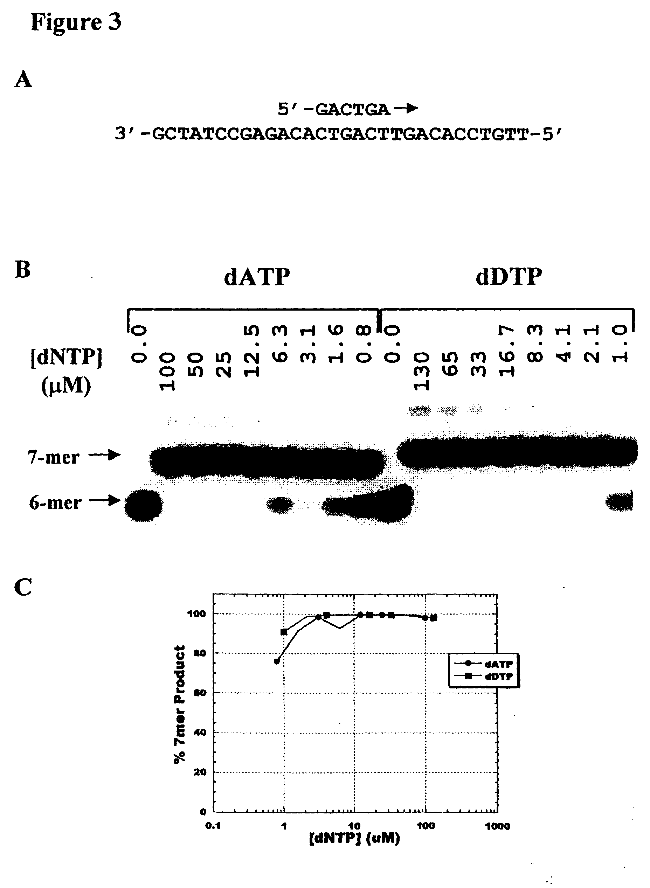

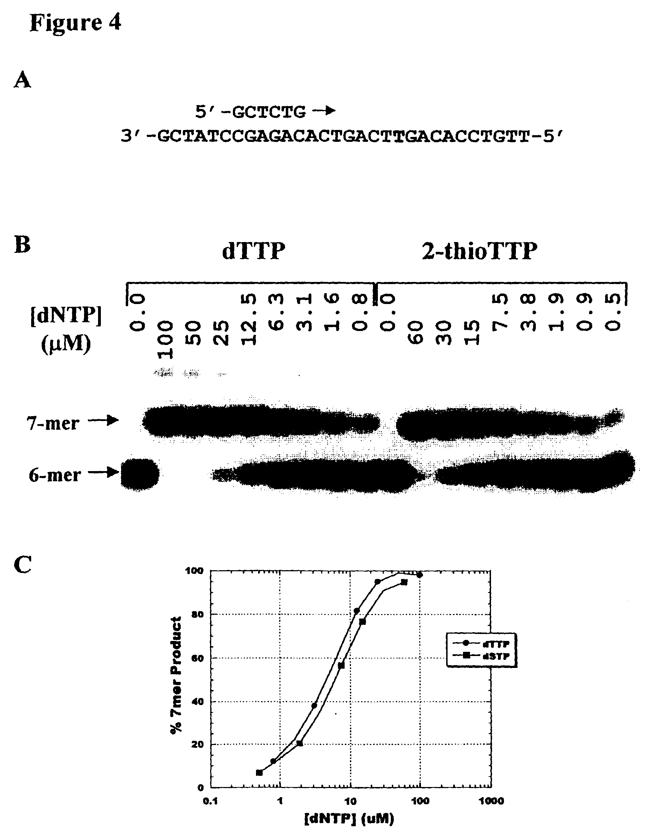

To further assess the incorporation efficiency of the modified nucleotides by Bst DNA polymerase, the efficiency for a single nucleotide extension reaction was determined using two 6-mer primers in the presence of varying concentrations of dATP, dDTP, dTTP or 2-thioTTP (Figure 3A and 4A). For this study, the reaction mixtures contained; 20 mM Tris-Cl (pH 8.8 @ 25° C), 10 mM KCl, 10 mM $(NH_4)_2SO_4$, 2 mM $MgSO_4$, 0.1% Triton-X100, 500 nanoM 6-mer primer, 20 nanoM 30-mer DNA template, 0.8 units/microL (~70 nanoM) Bst DNA polymerase and a dNTP concentration ranging from 0.5 microM to 130 microM. The reaction mixtures were incubated at 45° C for 8 hours and separated by electrophoresis on 20% denaturing (7M urea) polyacrylamide gels.

The GACTGA 6-mer primer is extended with dATP and dDTP (D) with approximately equal efficiencies (Figure 3B & 3C). Likewise, the extension efficiency of the GCTCTG 6-mer primer with the dTTP and 2-thioTTP (S) are also very similar and exhibit a 50% incorporation at about 4 and 6 microM respectively (Figure 4B & 4C). These results indicate that the dDTP and 2-thioTTP are both very good substrates for the Bst DNA polymerase and possess $k_{car}/K_m$ values near that of their natural counterparts.

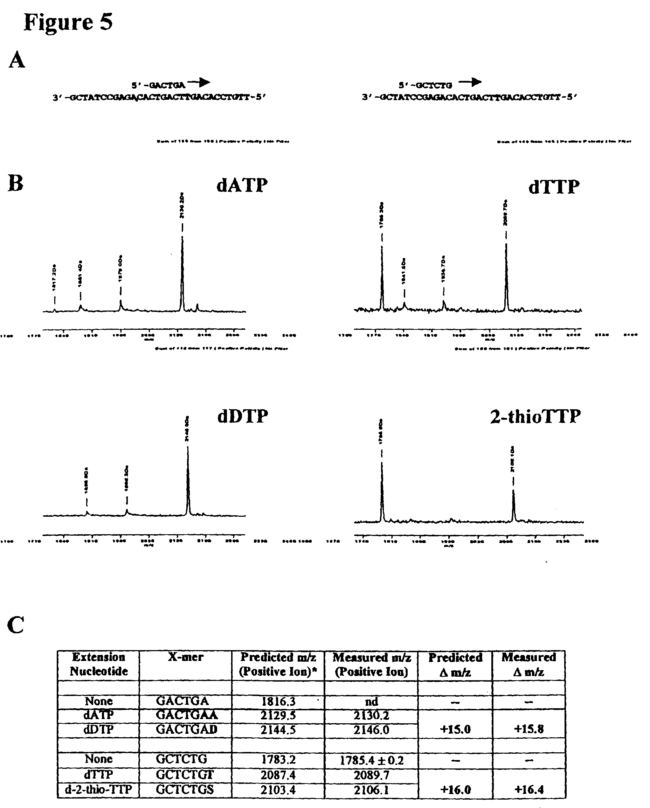

To establish that the extension products generated in the presence of the dDTP and 2-thioTTP were not due to contaminating dATP and dTTP in the dDTP and 2-thioTTP preparations, a mass spectroscopic analysis was performed on the extension reaction mixtures using the 6-mer primers (Figure 5A). The extension reactions were performed in 0.65ml pre-siliconized microfuge tubes containing the following components; 20 mM Tris-Cl (pH 8.8 @ 25° C), 10 mM KCl, 10 mM $(NH_4)_2SO_4$, 2 mM $MgSO_4$, 0.1% Triton-X100, 500 nM 6-mer primer, 20 nM 30-mer DNA template, 0.8 units/microL (~70 nM) Bst DNA polymerase and 20 microM dNTP. The reaction mixtures were incubated at 45° C for 4 hours and quenched with EDTA. 5 microL of the reaction mixture was mixed with 15 microL of distilled $H_2O$ and 20 microL of matrix solution (0.2 M 2,6-dihydroxyacetophenone, 0.2 M diammonium hydrogen citrate). One microliter samples were spotted and dried on a MALDI sample grid plate analyzed by Matrix Assisted Laser Desorption Ionization Time-of-Flight (MALDI-TOF) mass spectrometry.

The reaction mixtures containing the 6-mer primer GACTGA and either dATP or dDTP give single extension products having m/z values of 2130.2 and 2146.0 respectively (Figure 5B). This results in a 15.8 amu difference between the two 7-mer extension products which is consistent with the mass difference between the adenosine and the 2-aminoadenosine bases (Figure 5C). Likewise, the reaction mixtures containing the 6-mer primer GCTCTG and either dTTP or 2-thioTTP (S) give single extension reaction products having m/z values of 2089.7 and 2106.1 respectively. The resulting 16.4 amu difference between these two 7-mer extension products is consistent with the mass difference between the thymidine and 2-thiothymidine bases (Figure 5C). These results conclusively show that both the 2-aminoadenosine and 2-thiothymidine nucleotides triphosphates are indeed incorporated by the Bst DNA polymerase and that the dDTP and 2-thioTTP preparations do not contain any contaminating dATP and dTTP respectively;

**Example 3: Synthesis of Single Stranded Polynucleotides**

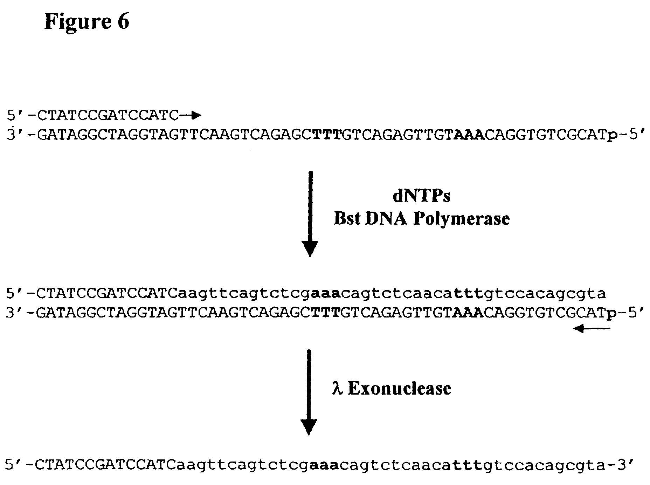

Unstructured single stranded UNA was generated by incorporating 2-aminoadenosine and 2-thiothymidine nucleotides using a 14-mer primer and 56-mer template (5'-phosphorylated) and Bst DNA Polymerase followed by digestion of the DNA template with Lambda Exonuclease (Figure 6). Ten microliter extension reactions were performed in 0.65ml pre-siliconized microfuge tubes containing the following components; 20 mM Tris-Cl (pH 8.8 @ 25° C), 10 mM KCl, 10 mM $(NH_4)_2SO_4$, 2 mM $MgSO_4$, 0.1% Triton-X100, 1.0 microM primer/template, 500 microM dGTP, 500 microM dATP (or dDTP), 500 microM dTTP (or 2-thioTTP ), 200 microM $^{32}$P- -CTP and 0.8 units/microL Bst DNA polymerase. The reactions were incubated at 65° C for 30 minutes, quenched with 5 mM EDTA, ethanol precipitated, dried and resuspended in 10 microL of 10 mM Tris-Cl pH 8.0. The 56-mer template was digested by incubating the resuspended samples with 10 units of lambda exonuclease (Strandase Kit™, (Novagen; Madison, WI)) for 30 minutes at 37° C. The reactions were quenched with 5 mM EDTA, ethanol precipitated, dried and resuspended in 10 microL of 10 mM Tris-Cl pH 8.0. The samples were then electrophoresed on 10% denaturing (7M urea) polyacrylamide gels and visualized using phosphorimaging methods.

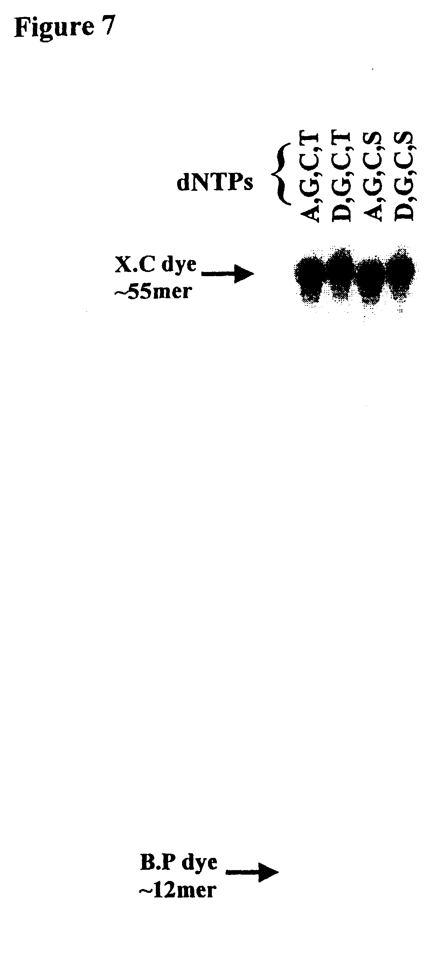

As shown in Figure 7, full-length 56-mer product is generated in the presence of either the four standard dNTPs or when dDTP (D) and 2-thioTTP (S) are substituted for dATP and dTTP respectively. In addition, little if any premature termination products are generated in the reactions containing dDTP and 2-thioTTP. Thus because the DNA template sequence includes three tandem A and T residues (Bold text in Figure 6), the results show that the Bst DNA polymerase can efficiently incorporate the 2-aminoadenosine and 2-thiothymidine nucleotides at adjacent sites in the polynucleotide product.

**Example 4: Preparative Synthesis and Spectral Characterization of Single Stranded Polynucleotide Sequences Containing the 2-aminoadenosine and 2-thiothymidine Nucleotides**

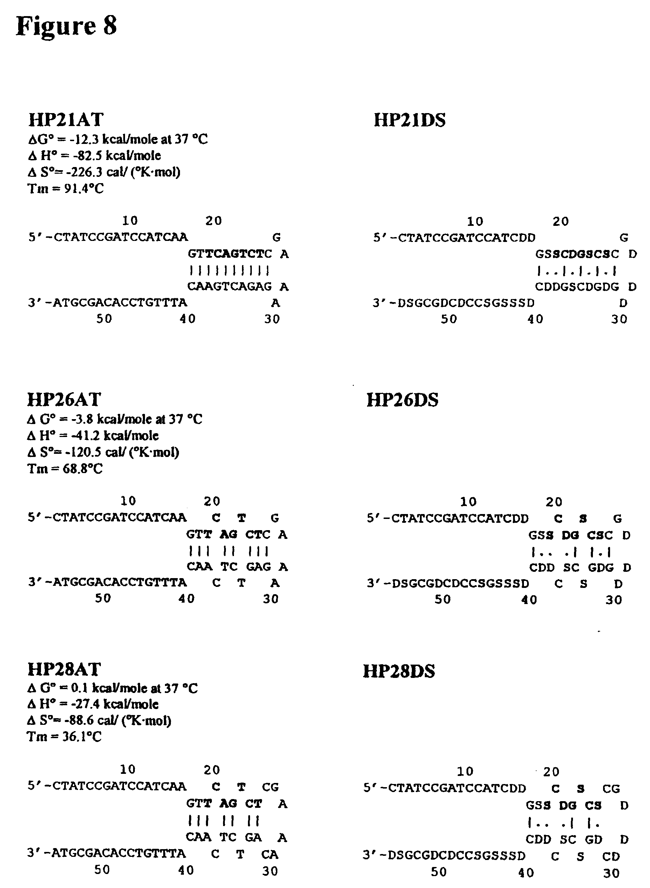

Three related 56-mer polynucleotide sequences were designed which are predicted to form hairpin stem-loop structures of various stability flanked by a common sequence which is predicted to be unstructured (Figure 8). The 56-mer HP21AT forms a 10 base-pair stem closed by a thermodynamically stable (C)GAAA(G) tetra-loop (Antao, et al., *Nucleic Acids Research*, 19; 5901-5905 (1991)) and has a predicted Tm of 91° C (SantaLucia, *Proc. Natl. Acad. Sci. USA*, 95; 1460-1465 (1998)). The 56-mer polynucleotides HP26AT and HP28AT are two and three nucleotide substitution variants of the HP21AT sequence which disrupt the stem structure and have predicted Tms of 69° C and 36° C respectively. HP21DS, HP26DS and HP28DS are the corresponding targets having the modified nucleotides 2-aminoadenosine (D) and 2-thiothymidine (S) which are expected to destabilize the stem structure by preventing stable A-T base-pairing.

Using the polymerase extension method described above, the HP21, HP26 and HP28 56-mer polynucleotide sequences were synthesized having either the four standard nucleotides or having the two modified nucleotides D and S in place of A and T. The polymerase extension reactions (250 uL) were performed in 0.65ml pre-siliconized microfuge tubes containing the following components; 20 mM Tris-Cl (pH 8.8 @ 25° C), 10 mM KCl, 10 mM $(NH_4)_2SO_4$, 2 mM $MgSO_4$, 0.1% Triton-X100, 2.0 microM 14-mer primer/56-mer (5'-phosphorylated) template, 500 microM dGTP, 500 microM dATP (or dDTP), 500 microM dTTP (or 2-thioTTP), and 0.8 units/microL Bst DNA polymerase. The reactions were incubated at 65° C for 45 minutes, quenched with 5 mM EDTA, ethanol precipitated, dried and resuspended in 50 microL of 10 mM Tris-Cl pH 8.0. The 56-mer template was digested by incubating the resuspended samples with 40 units of lambda exonuclease (Strandase kit™; Novagen, Madison, WI) for 45 minutes at 37° C. The reactions were quenched with 5 mM EDTA, ethanol precipitated, dried arid resuspended in 15 microL of 10 mM Tris-Cl pH 8.0. The samples were then electrophoresed on 10% semi-denaturing (3.5M urea) polyacrylamide gels. The polynucleotide bands were visualized by UV-shadowing, excised and eluted from the gel in buffer containing 20 mM Tris-Cl pH 7.6, 100 mM NaCl, precipitated with ethanol, and dried under vacuum. The polynucleotides were resuspended in 100 uL of 1.0 mM Tris-Cl (pH 7.6) and quantitated by UV absorbance assuming an extinction coefficient of $\varepsilon_{260} = 5.12 \times 10^5$ M$^{-1}$ for all six 56-mer polynucleotides.

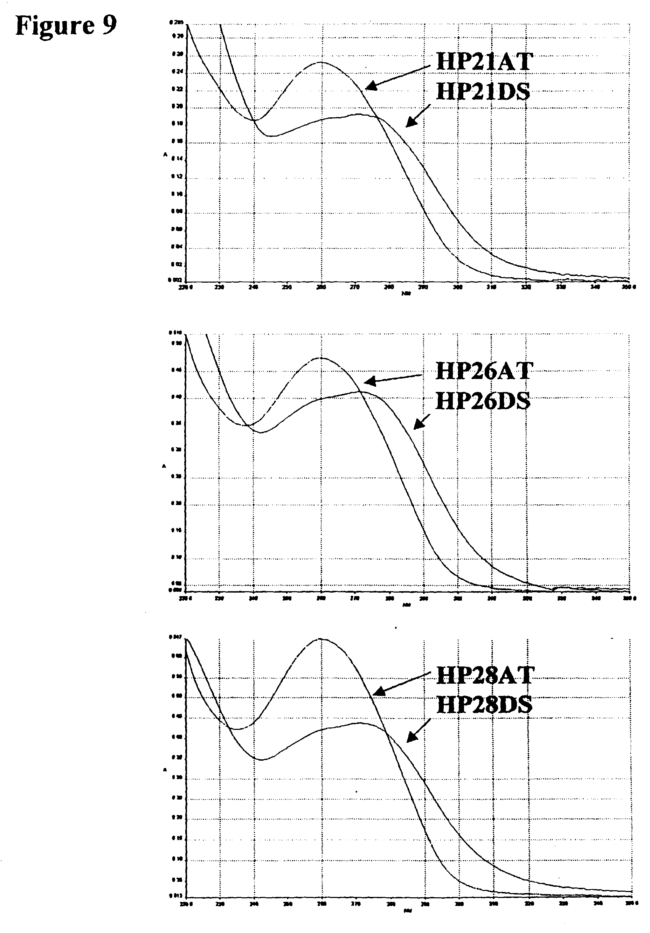

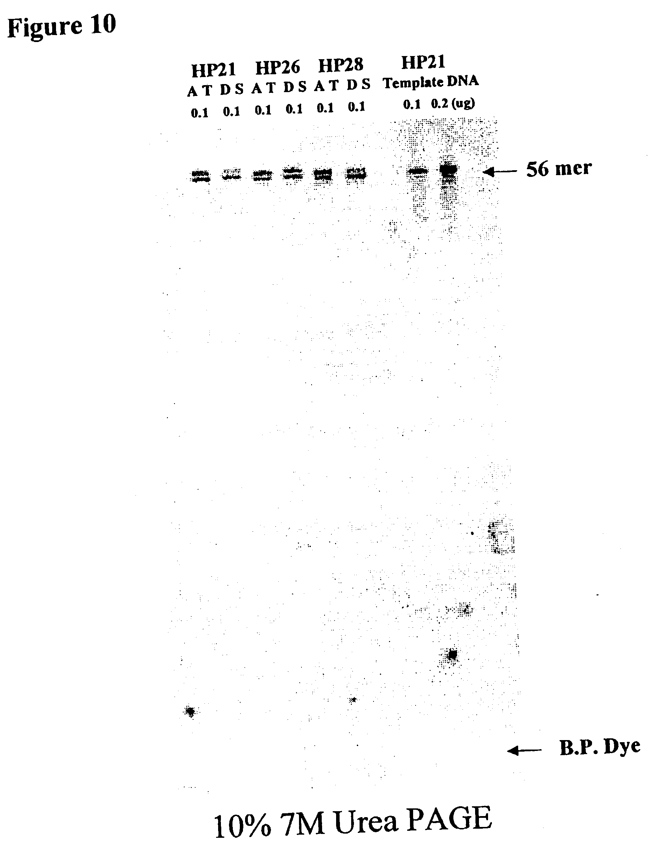

The three 56-mer polynucleotides containing the 2-aminoadenosine and 2-thiothymidine modified nucleotides show the expected red shift in $\lambda_{max}$ (Figure 9). This is due to the presence of the modified base 2-aminoadenosine which has two peaks; one at 259nm and one at 280nm and the 2-thiothymidine which has a $\lambda_{max}$ at about 276 nm. A small amount (0.1 microgram) of the six purified 56-mer polynucleotide samples were separated by denaturing PAGE and stained with Stains-All™ (Figure 10). The results show that all six 56-mer products are doublets that co-migrate with a 56 nucleotide single stranded DNA marker. These analyses confirm that the polymerase efficiently incorporates the two modified nucleotides into the polynucleotide sequences.

The UNA polynucleotides can be further purified using ion-exchange or reverse-phase chromatography. It was found that the polynucleotides containing the 2-aminoadenosine and 2-thiothymidine modified nucleotides do not efficiently elute from ion-exchange columns such as Elutip™ Minicolumns (Schleicher & Schuell) under high salt conditions (e.g. 1M NaCl) unless a small amount (10%) of an organic solvent such as acetonitrile is present in the elution buffer (data not shown). Consistent with this result, it was found that the modified polynucleotides elute at higher acetonitrile concentrations than their natural counterpart when analyzed by HPLC using C-18 reverse-phase columns and triethylammonium acetate buffers (data not shown). This overall increase in hydrophobic character could be due to either a direct chemical properties of the 2-aminoadenosine and 2-thiothymidine nucleotides or an increased exposure of the hydrophobic base resulting from the disruption of the natural secondary structure of the modified polynucleotides. Regardless of the specific mechanism responsible for this effect, it is suggested that all reaction vessels used for the synthesis, purification and assays of these types of UNAs be treated with a siliconizing agent to prevent non-specific binding of the UNAs to the reaction vessels.

### Example 5: Effect of the 2-aminoadenosine and 2-thiothymidine Nucleotides on Polynucleotide Secondary Structure; Polymerase Extension Assay

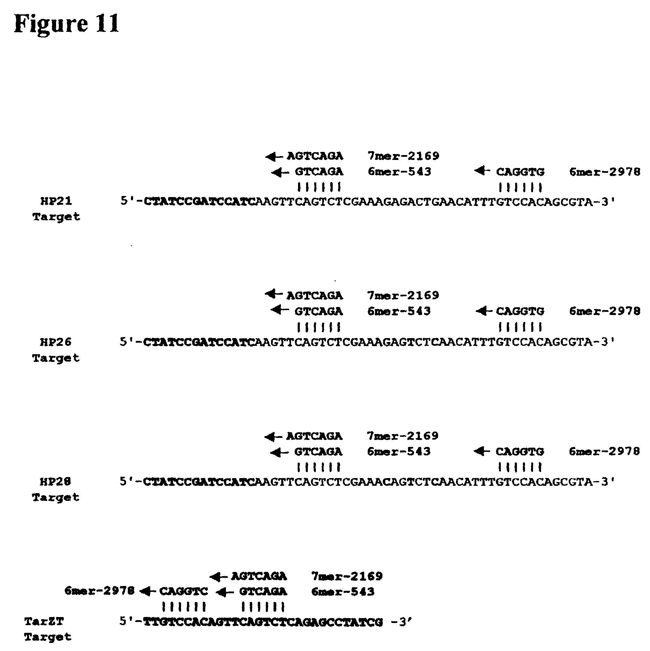

The effect of incorporating the 2-aminoadenosine and 2-thiothymidine nucleotides into the polynucleotides on the polynucleotide's structure was assessed by determining their relative ability to promote a single nucleotide polymerase extension reaction using short 6-mer and 7-mer oligonucleotides as the primers (Figure 11). The 6-mer-543 and 7-mer-2169 have their complementary binding site located within stem structure of each of the target polynucleotides HP21, HP26 and HP28 (see Figure 8, bold text). The complementary binding site for the 6-mer-2978 lies outside of the stem-loop structure and serves as a control primer. The single stranded target polynucleotide TarZT, which is predicted to have no secondary structure, served as a control target. All target sequences have a thymidine residue directly 5' to the primer binding sites which will direct the incorporation of a single ddATP residue into the primer sequence during the polymerase extension reaction.

The polymerase extension reactions (40 uL) were performed in 0.65ml pre-siliconized microfuge tubes containing the following components; 10 mM Tris-Cl (pH 8.4 @ 25 oC), 0.05% Triton X-100, 1.0 mM $MgCl_2$, 320 microM $MnCl_2$, 80 microM ddATP, 10 nM target polynucleotide, 100 nM primer ($^{32}$P-labeled on the 5' terminus @ S.A. 750 Ci/mmole) and 3.5 nM *Bacillus Stearothermophilus* (Bst) DNA polymerase. The reaction mixtures were incubated at 45° C, and 8 uL aliquots were removed at 3, 6 and 24 hours, quenched with EDTA, separated by electrophoresis on 20% denaturing PAGE, and visualized using phosphorimaging methods.

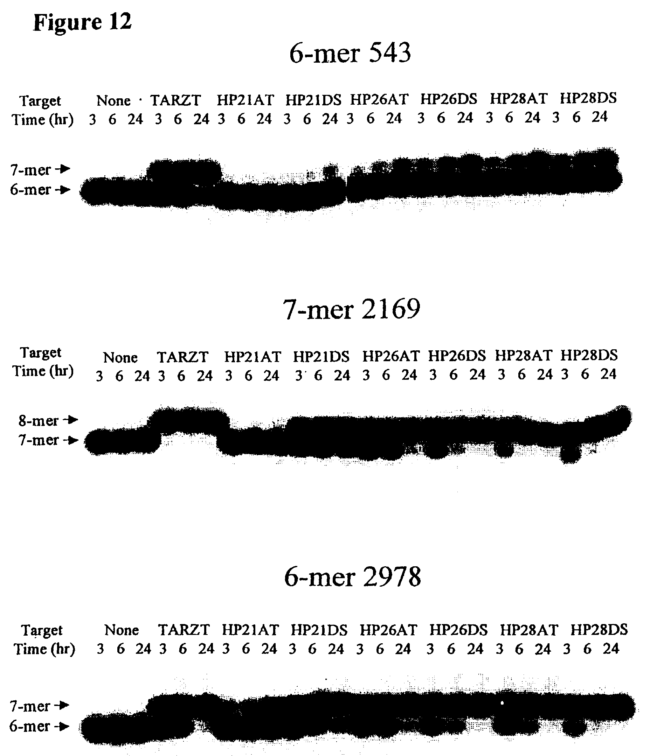

As shown in Figures 11 and 12, the 6-mer-543 is efficiently extended to the 7-mer product in the presence of the control polynucleotide TarZT. In contrast, no extension product is produced after 24 hours in the presence of the polynucleotide HP21AT. This is expected since the 6-mer-543 binding site is buried within the secondary structure of the polynucleotide and not available for hybridization with the 6-mer primer. Importantly however, a detectable level of 7-mer product is generated in the presence of the related polynucleotide HP21DS, which contains the modified nucleotides D and S. This result is even more dramatic for the single-base extension reactions using the 7-mer-2169. In the presence of the polynucleotide HP21AT, no 8-mer product is generated after 24 hours whereas in the presence of the modified polynucleotide HP21DS, greater than 60% of the 7-mer-2169 is converted to the 8-mer product after 24 hours. These same trends hold true for the other two pairs of polynucleotide targets having the two and three nucleotide substitutions. HP26DS is a better target than its related HP26AT polynucleotide and HP28DS is a slightly better target than HP28AT.

Interestingly, the D and S-containing polynucleotides are more efficient targets for the extension of the 6-mer-2978 whose binding site lies outside of the stem-loop structure. This could, be due to a greater stability of the primer/target duplex for the D and S-containing polynucleotides. It has been shown that A-S and T-D base-pairs are more stable than the standard A-T and T-A base-pairs (Kutyavin et al. 1996). Importantly however, there is a correlation between the predicted stability of the polynucleotide's secondary structure and its efficiency as a target in the single-base extension reaction. HP28 is a more efficient target than HP26 which, in turn, is a more efficient target than HP21 suggesting that intramolecular target structures near a primer binding site can effect the polymerase extension efficiency at that site. Thus because this same trend is exaggerated for the D and S-containing polynucletides, the results support the conclusion that the modifications do indeed alter the secondary structure of the polynucleotide targets. Regardless of the exact mechanism, these results clearly demonstrate that incorporating the 2-aminoadenosine and 2-thiothymidine nucleotide pair into a polynucleotide sequence increases the utility of the polynucleotide in hybridization-based assays.

**Claims**

1.    A method of producing an unstructured nucleic acid, comprising the steps of:

   i) providing a nucleic acid template including a first nucleic acid sequence and a second nucleic acid sequence substantially complementary to said first nucleic acid sequence;
   ii) providing nucleic acid precursors to produce said unstructured nucleic acid which is complementary with said first and second nucleic acid sequences, said nucleotide precursors being unable to hybridise with complementary nucleotide precursors; and
   iii) contacting said nucleic acid template and said nucleotide precursors with an enzyme under conditions such that said unstructured nucleic acid is produced.

2.    The method according to claim 1, wherein the nucleic acid precursors comprise at least two nucleotides capable of being incorporated enzymatically into a polynucleotide, and wherein said at least two nucleotides are unable to form intramolecular base pairs but can form intermolecular base pairs.

3.    The method of claim 1 or claim 2, wherein the sequences complementary to said first nucleic acid sequence and said one another.

4.    The method according to any one of the preceding claims, wherein step ii) comprises providing:

   a)   2-aminodeoxyadenosine 5'-triphosphate, and 2-thiodeoxythymidine 5'-triphosphate; or
   b)   2-aminodeoxyadenosine 5'-triphosphate, 2-thiodeoxythymidine 5'-triphosphate, deoxyguanosine 5'-triphosphate, and deoxycytidine 5'-triphosphate; or
   c)   inosine 5'-triphosphate, and pyrrolopyrimidine 5'-triphosphate;
   d)   2-aminodeoxyadenosine 5'-triphosphate, 2-thiodeoxythymidine 5'-triphosphate, deoxyinosine 5'-triphosphate, and deoxypyrrolopyrimidine 5'-triphosphate; or
   e)   deoxyguanosine 5'-triphosphate, and 2-thiodeoxycytidine; or
   f)   deoxyadenosine 5'-thiodeoxycytidine 5'-triphosphate, or
   g)   2-aminoadenosine, 2-thiothymidine inosine, and pyrrolopyrimidine; or
   h)   2-aminodeoxyadenosine 5'-triphosphate, 2-thiodeoxythymidine 5'-triphosphate, deoxyguanosine 5'-triphosphate, and 2-thiodeoxycytidine 5'-triphosphate; or
   i)   mixtures thereof.

5.    The method according to any one of the preceding claims, wherein said enzyme is selected from the group consisting of: RNA polymerase, DNA polymerase, reverse transcriptase, ribozymes, self-replicating RNA molecules and mixtures thereof.

6.    An unstructured nucleic acid with reduced secondary structure relative to a nucleic acid of substantially identical nucleotide sequence having naturally occurring bases, wherein the unstructure nucleic acid has at least two sequence elements that are complementary, wherein the complementary sequence elements do not form intramolecular base pairs, wherein at least one sequence element of at least two complementary sequence elements is capable of hybridizing to a substantially complementary sequence in another nucleic acid.

**7.** The unstructured nucleic acid of claim 6, wherein the nucleic acid is synthesized by an enzyme, and optionally the enzyme is selected from the group consisting of: RNA polymerase, DNA polymerase, reverse transcriptase, ribozymes and self-replicating RNA molecules.

**8.** The unstructured nucleic acid of claim 6 or claim 7, wherein the nucleic acid is at least 110 nucleotides in length.

**9.** The unstructured nucleic acid of claim 6, claim 7 or claim 8, wherein nucleic acid comprises 2-aminoadenine and 2-thiothymidine.

**10.** The unstructured nucleic acid of any one of claims 6 to 9 producible by the method of any one of claims 1 to 5.

# Figure 1

C - G

T- A

T - 2-aminoA

2-thioT - A

C - I

PyrroloPyr - G

2-thioT - 2-aminoA

PyrroloPyr - I

2-thioC - I

2-thioC - G

18

# Figure 2

**A**

5'-CGATAGGCTCTG →
3'-GCTATCCGAGACCCTGACTTGACACCTGTT-5'

**B**

# Figure 3

**A**

5′ -GACTGA→
3′ -GCTATCCGAGACACTGACTTGACACCTGTT-5′

**B**

| | dATP | dDTP |

[dNTP]
(μM)

dATP: 0.0 / 100, 50, 25, 12.5, 6.3, 3.1, 1.6, 0.8
dDTP: 0.0 / 130, 65, 33, 16.7, 8.3, 4.1, 2.1, 1.0

7-mer →
6-mer →

**C**

# Figure 4

**A**

5′-GCTCTG →
3′-GCTATCCGAGACACTGACTTGACACCTGTT-5′

**B**

**C**

# Figure 5

**A**

5'-GACTGA →
3'-GCTATCCGAGACACTGACTTGACACCTGTT-5'

5'-GCTCTG →
3'-GCTATCCGAGACACTGACTTGACACCTGTT-5'

**B**

dATP

dTTP

dDTP

2-thioTTP

**C**

| Extension Nucleotide | X-mer | Predicted m/z (Positive Ion)* | Measured m/z (Positive Ion) | Predicted Δ m/z | Measured Δ m/z |
|---|---|---|---|---|---|
| None | GACTGA | 1816.3 | nd | — | — |
| dATP | GACTGAA | 2129.5 | 2130.2 | | |
| dDTP | GACTGAD | 2144.5 | 2146.0 | +15.0 | +15.8 |
| None | GCTCTG | 1783.2 | 1785.4 ± 0.2 | — | — |
| dTTP | GCTCTGT | 2087.4 | 2089.7 | | |
| d-2-thio-TTP | GCTCTGS | 2103.4 | 2106.1 | +16.0 | +16.4 |

# Figure 6

5'-CTATCCGATCCATC→
3'-GATAGGCTAGGTAGTTCAAGTCAGAGC**TTT**GTCAGAGTTGT**AAA**CAGGTGTCGCATp-5'

<div align="center">↓ dNTPs<br>Bst DNA Polymerase</div>

5'-CTATCCGATCCATCaagttcagtctcg**aaa**cagtctcaacat**ttt**gtccacagcgta
3'-GATAGGCTAGGTAGTTCAAGTCAGAGC**TTT**GTCAGAGTTGT**AAA**CAGGTGTCGCATp-5'
                                                                    ←

<div align="center">↓ λ Exonuclease</div>

5'-CTATCCGATCCATCaagttcagtctcg**aaa**cagtctcaacat**ttt**gtccacagcgta-3'

# Figure 7

dNTPs { A,G,C,T  D,G,C,T  A,G,C,S  D,G,C,S

X.C dye ⟶
~55mer

B.P dye ⟶
~12mer

# Figure 8

## HP21AT

ΔG° = -12.3 kcal/mole at 37 °C
Δ H° = -82.5 kcal/mole
Δ S°= -226.3 cal/ (°K·mol)
Tm = 91.4°C

```
            10        20
5'-CTATCCGATCCATCAA              G
                GTTCAGTCTC  A
                ||||||||||
                CAAGTCAGAG  A
3'-ATGCGACACCTGTTTA            A
         50        40        30
```

## HP21DS

```
              10        20
5'-CTATCCGATCCATCDD              G
                  GSSCDGSCSC  D
                  |..|.|.|.|
                  CDDGSCDGDG  D
3'-DSGCGDCDCCSGSSSD            D
           50        40        30
```

## HP26AT

Δ G° = -3.8 kcal/mole at 37 °C
Δ H° = -41.2 kcal/mole
Δ S°= -120.5 cal/ (°K·mol)
Tm = 68.8°C

```
            10        20
5'-CTATCCGATCCATCAA     C   T   G
                GTT AG CTC  A
                ||| || |||
                CAA TC GAG  A
3'-ATGCGACACCTGTTTA     C   T   A
         50        40        30
```

## HP26DS

```
              10        20
5'-CTATCCGATCCATCDD     C   S   G
                  GSS DG CSC  D
                  |.. .| |.|
                  CDD SC GDG  D
3'-DSGCGDCDCCSGSSSD     C   S   D
           50        40        30
```

## HP28AT

Δ G° = 0.1 kcal/mole at 37 °C
Δ H° = -27.4 kcal/mole
Δ S°= -88.6 cal/ (°K·mol)
Tm = 36.1°C

```
            10        20
5'-CTATCCGATCCATCAA     C   T   CG
                GTT AG CT  A
                ||| || ||
                CAA TC GA  A
3'-ATGCGACACCTGTTTA     C   T   CA
         50        40        30
```

## HP28DS

```
              10        20
5'-CTATCCGATCCATCDD     C   S   CG
                  GSS DG CS  D
                  |.. .| |.
                  CDD SC GD  D
3'-DSGCGDCDCCSGSSSD     C   S   CD
           50        40        30
```

25

**Figure 9**

# Figure 10

HP21    HP26    HP28    HP21
A T D S  A T D S  A T D S  Template DNA
0.1  0.1  0.1  0.1  0.1  0.1   0.1  0.2 (ug)

←— 56 mer

←— B.P. Dye

## 10% 7M Urea PAGE

# Figure 11

```
                              ◄─ AGTCAGA      7mer-2169
                              ◄─ GTCAGA       6mer-543        ◄ CAGGTG    6mer-2978
                                 ||||||                          ||||||
HP21          5'-CTATCCGATCCATCAAGTTCAGTCTCGAAAGAGACTGAACATTTGTCCACAGCGTA-3'
Target
```

```
                              ◄─ AGTCAGA      7mer-2169
                              ◄─ GTCAGA       6mer-543        ◄─ CAGGTG   6mer-2978
                                 ||||||                          ||||||
HP26          5'-CTATCCGATCCATCAAGTTCAGTCTCGAAAGAGTCTCAACATTTGTCCACAGCGTA-3'
Target
```

```
                              ◄─ AGTCAGA      7mer-2169
                              ◄─ GTCAGA       6mer-543        ◄ CAGGTG    6mer-2978
                                 ||||||                          ||||||
HP28          5'-CTATCCGATCCATCAAGTTCAGTCTCGAAACAGTCTCAACATTTGTCCACAGCGTA-3'
Target
```

```
                         ◄─ AGTCAGA      7mer-2169
         6mer-2978 ◄─ CAGGTC  ◄─ GTCAGA      6mer-543
                      ||||||      ||||||
TarZT         5'-TTGTCCACAGTTCAGTCTCAGAGCCTATCG -3'
Target
```

**Figure 12**

## 6-mer 543

| Target | None · TARZT | HP21AT | HP21DS | HP26AT | HP26DS | HP28AT | HP28DS |
|--------|--------------|--------|--------|--------|--------|--------|--------|
| Time (hr) | 3 6 24 3 6 24 | 3 6 24 | 3 6 24 | 3 6 24 | 3 6 24 | 3 6 24 | 3 6 24 |

7-mer →
6-mer →

## 7-mer 2169

| Target | None TARZT | HP21AT | HP21DS | HP26AT | HP26DS | HP28AT | HP28DS |
|--------|------------|--------|--------|--------|--------|--------|--------|
| Time (hr) | 3 6 24 3 6 24 | 3 6 24 | 3' 6 24 | 3 6 24 | 3 6 24 | 3 6 24 | 3 6 24 |

8-mer →
7-mer →

## 6-mer 2978

| Target | None TARZT | HP21AT | HP21DS | HP26AT | HP26DS | HP28AT | HP28DS |
|--------|------------|--------|--------|--------|--------|--------|--------|
| Time (hr) | 3 6 24 3 6 24 | 3 6 24 | 3 6 24 | 3 6 24 | 3 6 24 | 3 6 24 | 3 6 24 |

7-mer →
6-mer →

**Figure 13**

6mer-543

7mer-2169

6mer-2978